# EUROPEAN PATENT SPECIFICATION

(11) **EP 3 955 903 B1**
(45) Date of publication and mention of the grant of the patent: **25.06.2025**
(21) Application number: 20790881.5
(22) Date of filing: 15.04.2020
(51) Int. Cl.: A61K 31/00, C07C 237/10, A61K 31/16, C07C 237/20, A61K 31/165, A61K 31/435, A61K 31/496, A61K 31/5377, A61K 31/65, A61K 31/7048, A61K 38/12, C07C 237/04, C07C 233/40, A61P 31/04, A61K 45/06, C07C 233/78, A61K 38/14, A61K 31/575

(54) **SMALL-MOLECULAR ADJUVANTS AND IMPLEMENTATIONS THEREOF**
KLEINMOLEKULARE ADJUVANZIEN UND IMPLEMENTIERUNGEN DAVON
ADJUVANTS À PETITES MOLÉCULES ET MODES DE RÉALISATION ASSOCIÉS

(30) Priority: 15.04.2019 IN 201941015155
(43) Date of publication of application: 23.02.2022
(73) Proprietor: Jawaharlal Nehru Centre For Advanced Scientific Research, Jakkur, Bangalore 560064 (IN)
(72) Inventor: HALDAR, Jayanta, Bangalore 560064 (IN); DHANDA, Geetika, Bangalore 560064 (IN)
(74) Representative: Lavoix
(86) International application number: PCT/IN2020/050358
(87) International publication number: WO 2020/213008

(56) References cited:
- WO-A1-01/09149
- WO-A1-2015/136311
- WO-A2-02/062755
- US-A1- 2017 144 969
- KASIM POPAJ ET AL: "Synthetic Analogues of Naturally Occurring Spider Toxins: Synthesis of 2-(Hydroxyphenyl)propanamides of Spermidine and Spermine", HELVETICA CHIMICA ACTA, VERLAG HELVETICA CHIMICA ACTA, HOBOKEN, USA, vol. 84, no. 4, 26 April 2001 (2001-04-26), pages 797 - 816, XP071267841, ISSN: 0018-019X, DOI: 10.1002/1522-2675(20010418)84:4<797::AID-HLCA797>3.0.CO;2-2
- SMITH HELEN K. ET AL: "Comparison of Resin and Solution Screening Methodologies in Combinatorial Chemistry and the Identification of a 100 nM Inhibitor of Trypanothione Reductase", JOURNAL OF COMBINATORIAL CHEMISTRY., vol. 1, no. 4, 1 July 1999 (1999-07-01), US, pages 326 - 332, XP093008936, ISSN: 1520-4766, DOI: 10.1021/cc990013c
- BLAGBROUGH ET AL: "Total synthesis of polyamine amide spider toxin argiotoxin-636 by a practical reductive alkylation strategy", TETRAHEDRON LETTERS, ELSEVIER, AMSTERDAM , NL, vol. 36, no. 51, 18 December 1995 (1995-12-18), pages 9393 - 9396, XP005917539, ISSN: 0040-4039, DOI: 10.1016/0040-4039(95)01994-S
- MOHINI M. KONAI ET AL.: "MEMBRANE ACTIVE PHENYLALANINE CONJUGATED LIPOPHILIC NORSPERMIDINE DERIVATIVES WITH SELECTIVE ANTIBACTERIAL ACTIVITY", J. MED. CHEM., vol. 57, no. 22, 21 October 2014 (2014-10-21), pages 9409 - 9423, XP055196327, Retrieved from the Internet <URL:https://pubs.acs.org/doi/pdf/10.1021/jm5013566> DOI: 10.1021/jm5013566
- ROWE R C, SHESKEY P J, OWEN S C: "Handbook of pharmaceutical excipients. Fifth edition REG:177/07", 30 November 2005, PHARMACEUTICAL PRESS , London , ISBN: 978-0-85369-618-6, article RAYMOND C ROWE; PAUL J SHESKEY; SIAN C OWEN: "Table of Contents; Handbook of Pharmaceutical Excipients", pages: i - v, XP009531816, 031835

## Description

### TECHNICAL FIELD

The present disclosure relates to the field of medicinal chemistry and more particularly to the development of select combinations as an effective therapy against resistant bacterial infections. The present disclosure also relates to small-molecular adjuvants, its stereoisomers, pharmaceutically acceptable salts, polymorphs, solvates and hydrates thereof, and to pharmaceutical compositions containing them.

### BACKGROUND

The rapid emergence of antimicrobial resistance (AMR) to a majority of antibiotics has resulted in multidrug-resistant superbugs, causing high morbidity and mortality. According to a recent report published by the World Health Organization (WHO), *Acinetobacter baumannii, Pseudomonas aeruginosa* and carbapenem-resistant, extended spectrum β-lactamase (ESBL) producing Enterobacteriaceae are listed as critical pathogens. This urgently calls for extensive research and development to combat infections caused by these multi drug-resistant Gram-negative bacteria (GNB).

Due to the deteriorating antibiotic pipeline and the sluggish development of novel antibiotics, there is a dire need of innovative strategies. One such promising strategy would be the repurposing of existing antibiotics, thereby reducing the legal and developmental challenges involved. This strategy has been exploited by several research groups and companies to tackle the problem of antibacterial resistance. A list of recently identified combinations can be found in this review (Tyers, M et al. Nat Rev Microbiol. 2019, 17, 141).

Combinations of β-lactam antibiotics with β-lactamase inhibitors have already been identified as an effective therapeutic strategy and some of these combinations are also in different phases of clinical trials (Antibiotics currently in Global Clinical Development. 2018 https://www.pewtrusts.org). Vabomere, a combination of meropenem (β-lactam antibiotic) and vaborbactum (β-lactamase inhibitor) was approved by the US FDA in August, 2017.

Vancomycin derivatives have been used in combination with β-lactam antibiotics like meropenem (Yarlagadda, V et al. ACS Infect Dis. 2018, 4, 1093). Combination therapy of glycopeptide antibiotics like telavancin with colistin, at sub-inhibitory concentrations have shown to increase their activity against Gram-negative pathogens (Hornsey, M et al. Antimicrob Agents Chemother. 2012, 56, 3080). Colistin analogues without the aliphatic chain have been used in combination with various antibiotics against Gram-negative bacteria. An example of this is SPR741, a colistin based cationic peptide developed by Spero therapeutics at Massachusetts (Corbett, D et al. Antimicrob Agents Chemother. 2017, 61, e00200-17).

Polymeric compounds have also displayed synergy with antibiotics like tetracycline against resistant Gram-negative bacteria (Uppu, DS et al. PLoS One. 2015, 10, e0126757). Thus, the combination approach has exhibited various positive results, exhibiting its therapeutic potency. Moreover, combination therapies with membrane-active agents like colistin, colistin analogues and OAK peptides have been able to potentiate antibiotics against resistant Gram-negative bacteria. This has set up a platform to develop membrane-perturbing molecules with simpler synthetic strategies (a limitation for most of the above-mentioned adjuvants) and use them in combination with obsolete antibiotics against MDR Gram-negative bacteria.

The combination therapies with membrane-active agents have already been tested, but none of them have been approved yet for treating infections. The major limitations of the adjuvants used in these combinations lie in the complexity of their structural design, the cost of their manufacture and *in-vivo* toxicity associated with them. Another challenge associated with some of the above-mentioned therapies involves the usage of an already existing antibiotic, colistin. This can lead to resistance development against colistin, which is the last resort to treat Gram-negative bacterial infections. Thus, using colistin as an adjuvant is not a very good idea as this antibiotic must be reserved for times of therapeutic crisis. Moreover, most of the membrane-targeting adjuvants which had been used until now exhibit antibacterial activity. This puts selection pressure on bacteria to develop resistance to these adjuvants. Mechanisms of resistance development to membrane-active agents have already been reported (Steinbuch, KB et al. Med. Chem. Commun. 2016, 7, 86). Colistin analogues, without the aliphatic chain exhibit mild to no antibacterial activity but they have other synthetic challenges associated with them. Moreover, these membrane-active agents have slight toxicity associated with them as well. WO 2015/136311 relates to polyamine conjugates, its isomers, prodrugs, and pharmaceutically acceptable salts thereof useful in the treatment, prevention, or suppression of diseases mediated by microbes.

### SUMMARY OF THE INVENTION

The present invention relates to a compound, its stereoisomers, pharmaceutically acceptable salts, polymorphs, solvates and hydrates thereof, which is selected from a group consisting of:

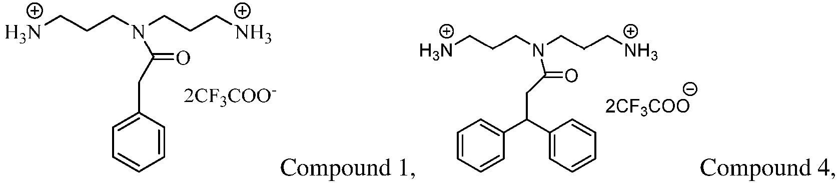

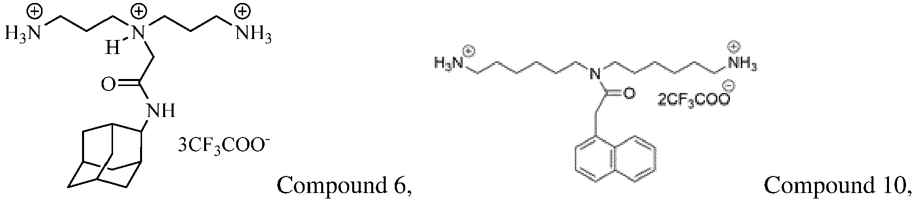

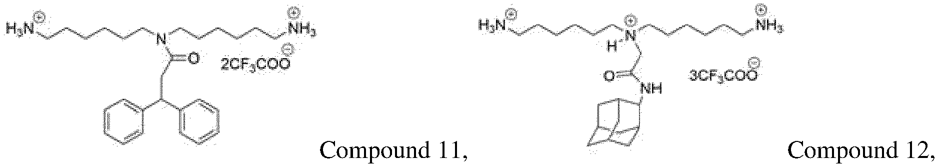

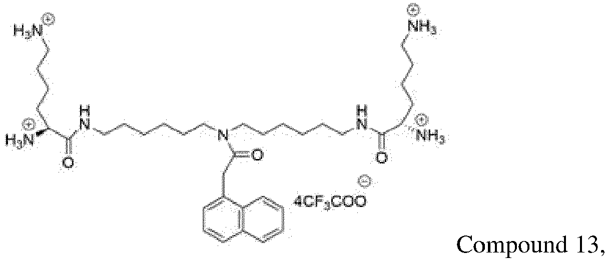

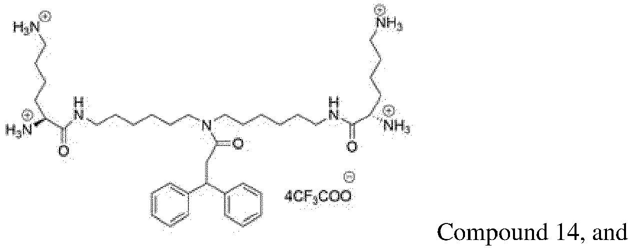

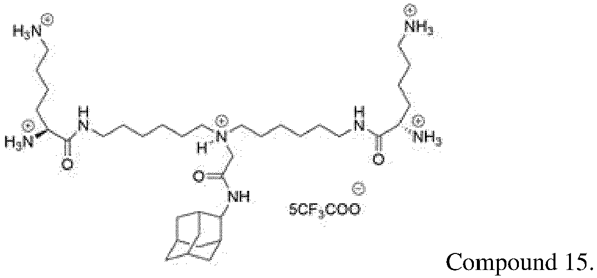

In another aspect of the present disclosure, there is provided the compounds of the present invention for use as small-molecular adjuvants.

In an aspect of the present disclosure, there is provided a pharmaceutical composition comprising the compounds of the present invention or its stereoisomers, pharmaceutically acceptable salts, polymorphs, solvates and hydrates thereof, together with a pharmaceutically acceptable carrier, optionally in combination with one or more other antibiotics, or their pharmaceutical composition.

In another aspect of the present disclosure, there is provided a pharmaceutical composition comprising: a) compound of the present invention or its stereoisomers, pharmaceutically acceptable salts, polymorphs, solvates and hydrates thereof as described herein; b) one or more antibiotics.

In one another aspect of the present disclosure, there is provided a pharmaceutical composition comprising: a) compound of the present invention or its stereoisomers, pharmaceutically acceptable salts, polymorphs, solvates and hydrates thereof as described herein; b) a pharmaceutical composition of one or more antibiotics.

In another aspect of the present disclosure, there is provided a use of a pharmaceutical composition as described herein, in treating a disease or condition in a patient, wherein said disease or condition is caused by microorganism selected from the group consisting of bacteria, fungi, and protozoa.

In yet another aspect of the present disclosure, there is provided a use of a pharmaceutical composition as to described herein, in killing or inhibiting the growth of a microorganism selected from the group consisting of bacteria, fungi, and protozoa.

These and other features, aspects and advantages of the present subject matter will be better understood with reference to the following description and appended claims. This summary is provided to introduce a selection of concepts in a simplified form.

### BRIEF DESCRIPTION OF THE DRAWINGS

The detailed description is described with reference to the accompanying figures. In the figures, the left-most digit(s) of a reference number identifies the figure in which the reference number first appears. The same numbers are used throughout the drawings to reference like features and components.

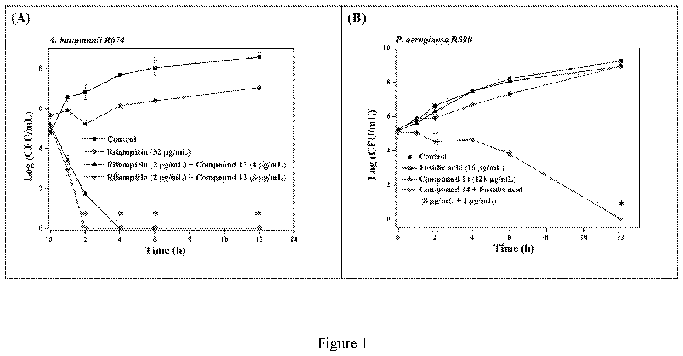

Figure 1 illustrates the bactericidal kinetics according to an implementation of the present disclosure. Figure 1(A) displays the bactericidal kinetics of the combination of rifampicin and Compound 13 and rifampicin against *A*. *baumannii* R674, whereas Figure 1(B) displays the bacterial kinetics of the combination of fusidic acid and Compound 14, Compound 14 and fusidic acid against *P. aeruginosa* R590; Asterisks correspond to <50 CFU/mL.

### DETAILED DESCRIPTION

Those skilled in the art will be aware that the present disclosure is subject to variations and modifications other than those specifically described. It is to be understood that the present disclosure includes all such variations and modifications. The disclosure also includes all such steps, features, compositions and compounds referred to or indicated in this specification, individually or collectively and any and all combinations of any or more of such steps or features.

### Definitions

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. Throughout this specification, unless the context requires otherwise the word "comprise", and variations, such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

The term "adjuvant" refers to a substance which is used in combination with a biological agent to enhance it's activity.

In the structural formulae given herein and throughout the present disclosure, the following terms have been indicated meaning, unless specifically stated otherwise.

Ratios, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a temperature range of about 100°C to about 200°C should be interpreted to include not only the explicitly recited limits of about 100°C to about 200°C, but also to include sub-ranges, such as 105°C to 155°C, 160°C to 175°C, and so forth, as well as individual amounts, including fractional amounts, within the specified ranges, such as 102.2°C, 150.6°C, and 171.3°C, for example.

In this specification, the prefix Cx-y as used in terms such as Cx-y alkyl, and the like (where x and y are integers) indicates the numerical range of carbon atoms that are present in the group; for example, C1-6 alkyl includes C1 alkyl (methyl), C2 alkyl (ethyl), C3 alkyl (propyl and isopropyl), and C4 alkyl (butyl, 1-methylpropyl, 2-methylpropyl, and t-butyl). Unless specifically stated, the bonding atom of a group may be any suitable atom of that group; for example, propyl includes prop-1-yl, and prop-2-yl.

Furthermore, the compound of the present invention, can be its derivatives, analogs, stereoisomers, diastereomers, geometrical isomers, polymorphs, solvates, co-crystals, intermediates, metabolites, or pharmaceutically acceptable salts and compositions.

The compounds of the present invention and their polymorphs, stereoisomers, solvates, co-crystals, intermediates, pharmaceutically acceptable salts, and metabolites thereof can also be referred as "compounds of the present disclosure".

The compounds according to the present invention, may contain one or more asymmetric centers (also referred to as a chiral centers) and may, therefore, exist as individual enantiomers, diastereoisomers, or other stereoisomeric forms, or as mixtures thereof. Chiral centers, such as chiral carbon atoms, may also be present in a substituent such as an alkyl group. Where the stereochemistry of a chiral center present in the compounds of the present invention, or in any chemical structure illustrated herein, is not specified, the structure is intended to encompass any stereoisomer and all mixtures thereof. Thus, compounds according to the present invention containing one or more chiral centers may be used as racemic modifications including racemic mixtures and racemates, enantiomerically-enriched mixtures, or as enantiomerically-pure individual stereoisomers.

Compounds disclosed herein include isotopes of hydrogen, carbon, oxygen, fluorine, chlorine, iodine and sulfur which can be incorporated into the compounds, such as not limited to ²H (D), ³H (T), c ¹¹C, ¹³C, ¹⁴C, ¹⁵N, ¹⁸F, ³⁵S, ³⁶Cl and ¹²⁵I. Compounds of this invention wherein atoms were isotopically labeled for example radioisotopes such as ³H, ¹³C, ¹⁴C, and the like can be used in metabolic studies and kinetic studies. Compounds of the invention where hydrogen is replaced with deuterium may improve the metabolic stability and pharmacokinetics properties of the drug such as in vivo half-life.

Individual stereoisomers of a compound according to the present invention which contain one or more asymmetric centers may be resolved by methods known to those skilled in the art. For example, such resolution may be carried out (1) by the formation of diastereoisomeric salts, complexes or other derivatives; (2) by selective reaction with a stereoisomer-specific reagent, for example by enzymatic oxidation or reduction; or (3) by gas-liquid or liquid chromatography in a chiral environment, for example, on a chiral support such as silica with a bound chiral ligand or in the presence of a chiral solvent. It will be appreciated that where the desired stereoisomer is converted into another chemical entity by one of the separation procedures described above, a further step is required to liberate the desired form.

Alternatively, specific stereoisomers may be synthesized by asymmetric synthesis using optically active reagents, substrates, catalysts or solvents, or by converting one enantiomer to the other by asymmetric transformation.

It is to be understood that the references herein to compounds of the present invention and salts thereof covers the compounds of the present invention as free bases, or as salts thereof, for example as pharmaceutically acceptable salts thereof. Thus, in one embodiment, the invention is directed to compounds of the present invention as the free base. In another embodiment, the invention is directed to compounds of the present invention and salts thereof. In a further embodiment, the invention is directed to compounds of the present invention and pharmaceutically acceptable salts thereof.

It will be appreciated that pharmaceutically acceptable salts of the compounds according to the present invention, may be prepared. Indeed, in certain embodiments of the invention, pharmaceutically acceptable salts of the compounds according to the present invention may be preferred over the respective free base because such salts impart greater stability or solubility to the molecule thereby facilitating formulation into a dosage form. Accordingly, the invention is further directed to compounds of the present invention, and pharmaceutically acceptable salts thereof.

Included within the scope of the "compounds of the invention" are all solvates (including hydrates), complexes, polymorphs, and stereoisomers, and salts thereof.

The compounds of the invention may exist in solid or liquid form. In the solid-state, the compounds of the invention may exist in crystalline or non-crystalline form, or as a mixture thereof. For compounds of the invention that are in crystalline form, the skilled artisan will appreciate that pharmaceutically acceptable solvates may be formed wherein solvent molecules are incorporated into the crystalline lattice during crystallization. Solvates may involve nonaqueous solvents such as ethanol, isopropyl alcohol, dimethylsulfoxide (DMSO), acetic acid, ethanolamine, and ethyl acetate, or they may involve water as the solvent that is incorporated into the crystalline lattice. Solvates wherein water is the solvent that is incorporated into the crystalline lattice are typically referred to as "hydrates". Hydrates include stoichiometric hydrates as well as compositions containing variable amounts of water. The invention includes all such solvates.

It will be further appreciated that certain compounds of the invention that exist in crystalline form, including the various solvates thereof, may exhibit polymorphism (i.e. the capacity to occur in different crystalline structures). These different crystalline forms are typically known as "polymorphs". The invention includes such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. It will be appreciated that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions. The term "polymorphs" refers to crystal forms of the same molecule, and different polymorphs may have different physical properties such as for example, melting temperatures, heats of fusion, solubilities, dissolution rates and/or vibrational spectra as a result of the arrangement or conformation of the molecules in the crystal lattice.

The term "substituted" in reference to a group indicates that a hydrogen atom attached to a member atom within a group is replaced. It should be understood that the term "substituted" includes the implicit provision that such substitution be in accordance with the permitted valence of the substituted atom and the substituent and that the substitution results in a stable compound (i.e. one that does not spontaneously undergo transformation such as rearrangement, cyclisation, or elimination). In certain embodiments, a single atom may be substituted with more than one substituent as long as such substitution is in accordance with the permitted valence of the atom. Suitable substituents are defined herein for each substituted or optionally substituted group.

The term "alkyl" refers to a saturated hydrocarbon chain having the specified number of carbon atoms. For example, which is not limited, C₁₋₆ alkyl refers to an alkyl group having from 1 - 6 carbon atoms, or 1 - 4 carbon atoms. Alkyl groups may be straight or branched chained groups. Representative branched alkyl groups have one, two, or three branches. Preferred alkyl groups include, without limitation, methyl, ethyl, n-propyl, isopropyl, butyl, isobutyl, and t-butyl.

The term "alkoxy" refers to an alkyl group attached via an oxygen linkage to the rest of the molecule. For example, C₁₋₆ alkoxy refers to an alkyl group having from 1 - 6 carbon atoms, or 1 - 4 carbon atoms attached via an oxygen linkage to the rest of the molecule. Preferred alkoxy groups include, without limitation, -OCH₃ (methoxy), -OC₂H₅ (ethoxy) and the like.

The term "haloalkyl" refers to a halogen in an alkyl group as defined above attached via alkyl linkage to the rest of the molecule. For example, C₁₋₆ haloalkyl refers to an alkyl group having from 1 - 6 carbon atoms, or 1 - 4 carbon atoms wherein one or more hydrogen atoms are replaced by the same number of identical or different halogen atoms. Preferred haloalkyl groups include, without limitation, -CH₂Cl, -CHCl₂, trifluoromethyl, 2,2,2-trifluoroethyl, and the like.

The term "haloalkoxy" refers to a halogen in an alkoxy group as defined above attached via alkoxy linkage to the rest of the molecule. For example, C₁₋₆ haloalkoxy refers to an alkoxy group having from 1 - 6 carbon atoms, or 1 - 4 carbon atoms wherein one or more hydrogen atoms are replaced by the same number of identical or different halogen atoms. Preferred haloalkoxy groups include, without limitation, -OCH₂Cl, -OCHCl₂, and the like

The term "halo" or "halogen" refers to a halogen radical, for example, fluoro, chloro, bromo, or iodo.

The term "cycloalkyl" refers to a saturated hydrocarbon ring having a specified number of carbon atoms, which may be monocyclic or polycyclic. For example, which is not limited, C₃₋₁₅ cycloalkyl refers to a cycloalkyl group having from 3 to 15 member atoms. The polycyclic ring denotes hydrocarbon systems containing two or more ring systems with one or more ring carbon atoms in common i.e. a spiro, fused or bridged structures. For example, which is not limited, C₃₋₁₅ cycloalkyl refers to a cycloalkyl group having from 3 to 15 membered atoms. Preferred cycloalkyl groups include, without limitation, cyclopropyl, cyclobutyl, cyclopentyl, cyclohexyl, cyclooctanyl, perhydronaphthyl, adamantyl, noradamantyl and norbornyl groups, bridged cyclic groups or spirobicyclic groups e.g spiro [4.4] non-2-yl, and the like.

The term "aryl" refers to an aromatic ring having a specified number of carbon atoms. For example, C₆₋₁₅ aryl refers to an aryl group having 6 to 15 member atoms, or 6 member atoms. Preferred aryl groups include, without limitation, phenyl, naphthyl, and the like.

The term "heteroaryl" refers to aromatic rings containing from 1 to 5 heteroatoms in the ring. "Heteroaryl" groups may be substituted with one or one or more substituents if so defined herein. The "C₁₋₆ heteroaryl" rings having 1 or 6 carbon as member atoms. The term "5-15 membered heteroaryl" has 1 to 15 carbon(s) as member atoms. The "heteroaryl" includes pyridinyl, tetrazolyl, pyrazolyl, or azacyclics, such as carbazole, indole, phenothiazine, and the like. "Heteroatom" refers to a nitrogen, sulfur, or oxygen atom, for example, a nitrogen atom or an oxygen atom.

The term "heterocyclyl" refers to saturated or unsaturated monocyclic aliphatic rings containing 5, 6, or 7 ring members including 1-3 heteroatoms or saturated or unsaturated bicyclic, tricyclic, tetracyclic aliphatic rings containing 5, 6 or 7 ring members including 1-3 heteroatoms, which may include spiro, fused, or bridged ring systems. In certain embodiments, "heterocyclyl" groups are saturated. In other embodiments, "heterocyclyl" groups are unsaturated. "Heterocyclyl" groups containing more than one heteroatom may contain different heteroatoms. "Heterocyclyl" groups may be substituted with one or more substituents as defined herein. The "C₁₋₁₀ heterocyclyl" refers to rings having 1 or 10 carbon as member atoms. "Heterocyclyl" includes piperidinyl, tetrahydropyranyl, azepinyl, oxazepinyl, azabicyclo[3.1.0]hexanyl.

The phrase "pharmaceutically acceptable" refers to those compounds, materials, compositions, and dosage forms which are, within the scope of sound medical judgment, suitable for use in contact with the tissues of human beings and animals without excessive toxicity, irritation, or other problem or complication, commensurate with a reasonable benefit/risk ratio.

The phrase "at least one protecting group" refers to protecting groups known in the art. Examples of many of these possible groups may be found in "Protective Groups in Organic Synthesis" by T.W. Green, John Wiley and Sons, 1981. Non-limiting examples for the protecting group may be tert-butoxycarbonyl (Boc or t-Boc), carbobenzoxy (Z or Cbz), 9-fluorenylmethoxycarbonyl (Fmoc), 9-(R)₃Si-Fmoc, Xmoc, DHAmoc, their derivatives, and the like.

As used herein, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free base form with a suitable acid.

Salts and solvates having non-pharmaceutically acceptable counter-ions or associated solvents are within the scope of the present invention, for example, for use as intermediates in the preparation of other compounds of the present invention, and their pharmaceutically acceptable salts. Thus, one embodiment of the invention embraces compounds of the present invention, and salts thereof. Compounds according to the present invention, contain a basic functional group and are therefore capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, trifluoroacetate, hydroxyacetate, phenyl acetate, propionate, butyrate, iso-butyrate, valerate, maleate, hydroxymaleate, acrylate, fumarate, formate, malate, tartrate, citrate, salicylate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, naphthoate, hydroxynaphthoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), aminobenzenesulfonate, p- toluenesulfonate (tosylate), and naphthalene-2-sulfonate.

As discussed in the background section, the identification and development of simple, non-active and non-toxic adjuvants which can be used in combination with a number of antibiotics remains an area of immense interest. The present disclosure thus provides small molecular adjuvants which exhibit potentiation of antibiotics.

A term once described, the same meaning applies for it, throughout the disclosure.

There is provided a compound, or its stereoisomers, pharmaceutically acceptable salts, polymorphs, solvates and hydrates thereof, which is selected from a group consisting of

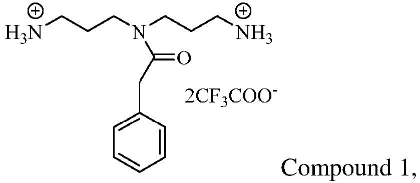

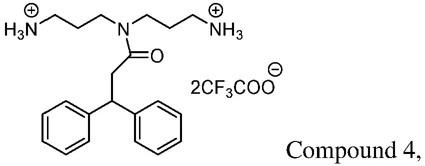

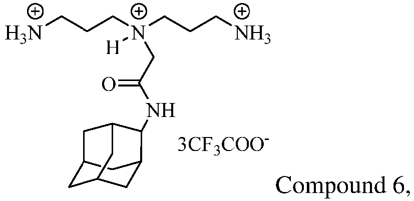

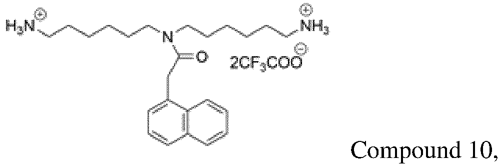

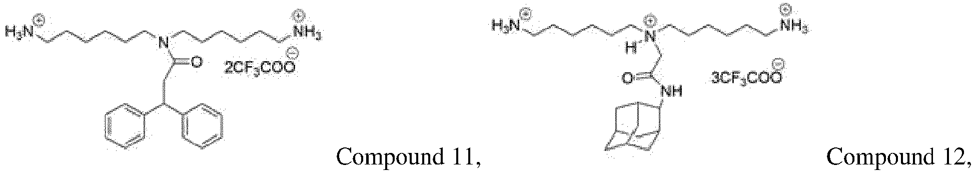

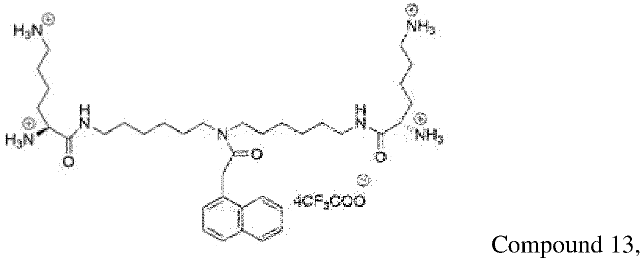

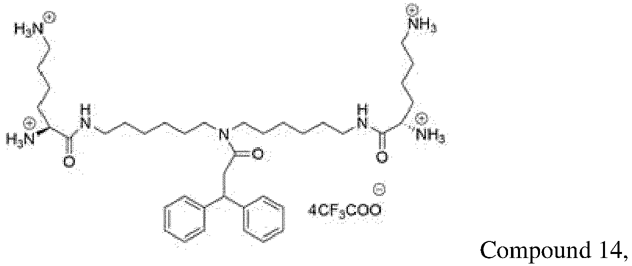

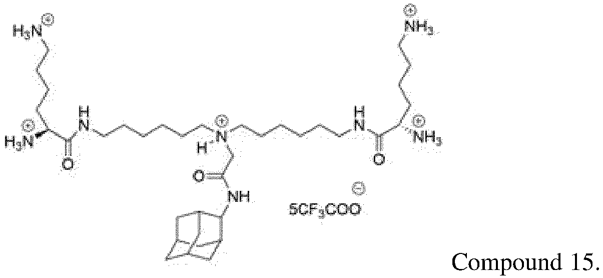

In an embodiment of the present disclosure, there is provided a compound, or its stereoisomers, pharmaceutically acceptable salts, polymorphs, solvates and hydrates thereof, which is selected from a group consisting of

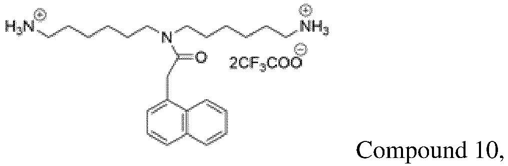

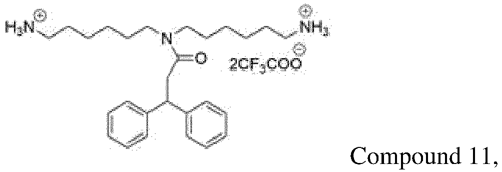

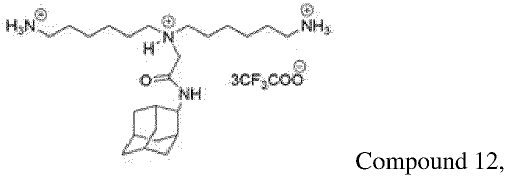

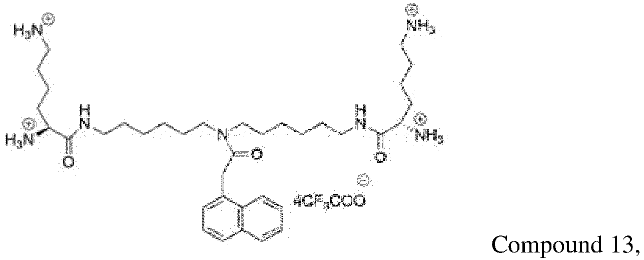

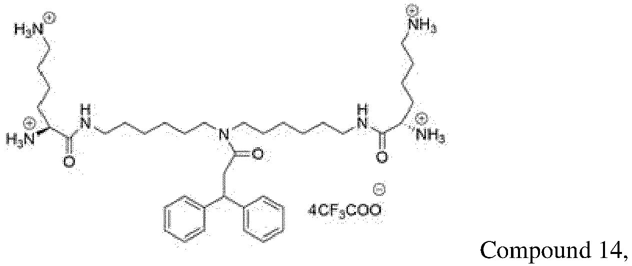

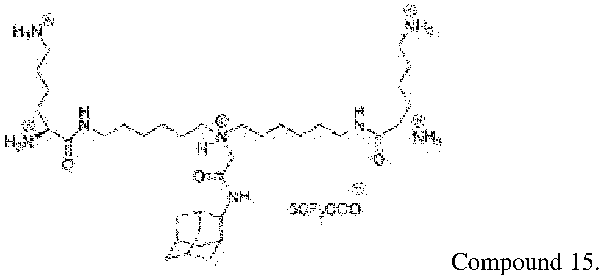

In an embodiment of the present disclosure, there is provided the compound of the present invention as described herein, for use as small molecular adjuvants.

In an embodiment of the present disclosure, there is provided a pharmaceutical composition comprising a compound of the present invention or its stereoisomers, pharmaceutically acceptable salts, polymorphs, solvates and hydrates thereof as described herein, together with a pharmaceutically acceptable carrier, optionally in combination with one or more other antibiotics, or their pharmaceutical composition.

In an embodiment of the present disclosure, there is provided a pharmaceutical composition comprising: a) compound of the present invention or its stereoisomers, pharmaceutically acceptable salts, polymorphs, solvates and hydrates thereof, as described herein; b) one or more antibiotics.

In an embodiment of the present disclosure, there is provided a pharmaceutical composition comprising: a) compound of the present invention or its stereoisomers, pharmaceutically acceptable salts, polymorphs, solvates and hydrates thereof, as described herein; b) pharmaceutical composition of one or more antibiotics.

In an embodiment of the present disclosure, there is provided a pharmaceutical composition as described herein, wherein the antibiotic is selected from the group consisting of rifampicin, tetracycline, fusidic acid, oxazolidinone, glycopeptides, fluoroquinolone, macrolide, beta-lactams, aminoglycosides, chloramphenicol, polymyxin, lipopeptide, and combinations thereof.

In an embodiment of the present disclosure, there is provided a pharmaceutical composition as described herein, wherein the antibiotic is selected from the group consisting of rifampicin, tetracycline, minocycline, fusidic acid, linezolid, vancomycin, ciprofloxacin, erythromycin, ampicillin, streptomycin, chloramphenicol, colistin, daptomycin, and combinations thereof.

In an embodiment of the present disclosure, there is provided a pharmaceutical composition as described herein, for use in treating disease or condition in a patient, wherein said disease or condition is caused by a microorganism selected from the group consisting of bacteria, fungi and protozoa.

In an embodiment of the present disclosure, there is provided a pharmaceutical composition as described herein, for use in killing or inhibiting the growth of a microorganism selected from the group consisting of bacteria, fungi, and protozoa.

In an embodiment of the present disclosure, there is provided a use of the pharmaceutical composition as described herein, in treating a disease or condition in a patient, wherein said disease or condition is caused by microorganism selected from the group consisting of bacteria, fungi, and protozoa.

In an embodiment of the present disclosure, there is provided a use of the pharmaceutical composition as described herein, in killing or inhibiting the growth of a microorganism selected from the group consisting of bacteria, fungi, and protozoa.

In an embodiment of the present disclosure, there is provided a pharmaceutical composition as described herein, wherein the bacteria is Gram-positive bacteria or Gram-negative bacteria.

In an embodiment of the present disclosure, there is provided a pharmaceutical composition as described herein, wherein the bacteria is a multi-resistant bacterium selected from a group consisting of *A*. *baumannii, P. aeruginosa, E. coli, K. pneumoniae,* or any combinations thereof.

### EXAMPLES

The disclosure will now be illustrated with working examples, which is intended to illustrate the working of disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein. It is to be understood that this disclosure is not limited to particular methods, and experimental conditions described, as such methods and conditions may apply.

The following examples provide the details about the synthesis, characterization and combination efficacies of the small-molecular adjuvants mentioned in the present invention.

All the solvents were of reagent grade, which were distilled and dried before its use. All the reagents were purchased from Sigma-Aldrich, Alfa-Aesar, S.D. Fine, Avra and Spectrochem, and were used without further purification. Analytical thin layer chromatography (TLC) was performed on E. Merck TLC plates pre-coated with silica gel 60 F₂₅₄ and visualization was carried out using UV light and Iodine. Column chromatography was performed on silica gel (60-120 mesh) using different ratio of chloroform and methanol solvent system. Nuclear magnetic resonance spectra were recorded on Bruker (AV-400) 400 MHz spectrometer in deuterated solvents. Mass spectra were obtained using 6538-UHD Accurate mass Q-TOF LC-MS instrument. For optical density measurement during bacteriology experiments, Tecan Infinite Pro series M200 Microplate Reader was used. Bacterial strains, *A*. *baumannii* (R674), *P. aeruginosa* (R590), *E. coli* (R3336) and *K. pneumoniae* (R3934) were used for the studies.

The combination efficacy with antibiotics from different classes and individual antibacterial activity of the synthesized adjuvants was evaluated against the above mentioned MDR and NDM-1 strains of Gram-negative bacteria. All the bacteria were grown in peptone. The bacterial samples were freeze dried and stored at -80°C. 3 µl of these stocks were added to 3 mL of peptone and the culture was grown for 6 h at 37°C prior to the experiments.

### Example 1: Synthesis of Compounds 1-5: (not part of the present invention)

Small-molecular adjuvants (SMAs) where R₁ = R₂ = Hydrogen and R₃ = Aromatic radicals were synthesized from *N¹*-Boc-*N*³-(3-(Boc-amino)-propyl) propane-1,3-diamine and different aromatic radical bearing carboxylic acids or amino acids, through simple amide coupling reaction using O-benzotriazole-N,N,N',N'-tetramethyl-uronium-hexafluorophosphate (HBTU) as coupling agent followed by deprotection using trifluoroacetic acid to give the final compounds with CF₃COO⁻ as counterions. The final compounds can be synthesized with other counterions such as Cl⁻, Br⁻ etc.

### Synthesis of N¹-Boc-N³-(3-(Boc-amino)propyl)propane-1,3-diamine (5a):

10 g (1 equivalent, 76.27 mmol) of norspermidine was dissolved in MeOH (50 mL) and the solution was kept at -80°C. Then 24.94 g (1.5 equivalents, 114.31 mmol) of Boc₂O was dissolved in MeOH (50 mL) and added to the reaction mixture dropwise. The reaction was continued for 1 h at -80°C. Then the reaction mixture was allowed to come at RT. MeOH was removed and purification was done through column chromatography on silica gel (60-120 mesh) using methanol and chloroform (7:93) as eluent to afford the product with 50% yield. ¹H-NMR (400 MHz, CDCl₃) δ/ppm: 5.182 (s, NH(-CH₂-CH₂-CH₂-*NH*Boc)₂, 2H), 3.190-3.175 (m, NH(-C*H*₂-CH₂-CH₂-NHBoc)₂, 4H), 2.649-2.616 (m, NH(-CH₂-CH₂-C*H*₂-NHBoc)₂, 4H), 1.860 (s, *NH*(-CH₂-CH₂-CH₂-NHBoc)₂, 1H), 1.666-1.602 (m, NH(-CH₂-C*H₂*-CH₂-NH-Boc)₂, 4H), 1.417 (s, NH(-CH₂-CH₂-CH₂-NH-COO-C(C*H₃*)*₃*)₂, 18H). HRMS (m/z): 332.2539 [(M+H)⁺] (Observed), 332.2549 [(M+H)⁺] (Calculated).

### Synthesis of N-Boc-L-Tryptophan (BocTrp):

4.7 g (1 equivalent, 23 mmol) of ^{L}Trp was dissolved in 50 mL water, 3.9 g (2 equivalents, 46 mmol) of NaHCO₃ was added and the reaction mixture was allowed to stir for 15-20 minutes. The reaction mixture was kept at 0⁰C and 6 g (1.2 equivalents, 27.6 mmol) of di-t-butylpyrocarbonate (Boc₂O), dissolved in 50 mL of tetrahydrofuran (THF) was added to the reaction mixture dropwise. The ice-bath was removed and the reaction was allowed to happen for 16-24 hours. Then, THF was evaporated and the reaction mixture was acidified using 1N hydrochloric acid (HCl) till a pH of 3-4 was attained. The precipitate obtained after acidifiacton was extracted using dichloromethane (DCM) to afford the pure product with 89% yield. ¹H-NMR (400 MHz, DMSO-d₆) δ/ppm: 12.509 (s, *-COOH,* 1H), 10.812 (s, -N*H*_{indole}, 1H), 7.527-6.925 (m, Ar*H* and -N*H*Boc, 6H), 4.174-4.119 (m, -C*H*NHBoc, 1H), 3.152-2.943 (m, *-CH₂,* 2H), 1.329 (s, -NH-COO-C(C*H₃)₃*, 9H).

### General procedure for synthesizing 5b- 5f:

About 1.2 equivalents of carboxylic acids with aromatic radicals (phenylacetic, naphthylacetic, biphenyl, diphenylpropionic acid) or Boc-protected tryptophan were dissolved in dry DCM (12 mL) at 0°C. In the reaction mixture, 3 equivalents of DIPEA was added followed by 1.2 equivalents of HBTU. Then DMF (3 mL) was added to the reaction mixture. After 10 minutes, 1 equivalent of **5a** in dry DCM (1 mL) was added dropwise to the reaction mixture. The reaction mixture was brought to RT and allowed to stir for 24 h. Solvent was evaporated and the residue was diluted in ethyl acetate (50 mL). Then work-up was carried out at first with 1N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as an eluent to afford **5b-5f** with 55-80% yield.

### N,N-bis(3-(Boc-amino)propyl) phenyl ethanamide (5b):

Yield-56%; ¹H-NMR (400 MHz, CDCl₃) δ/ppm: 7.333-7.297 (m, Ar*H*, 2H), 7.251-7.232 (m, Ar*H*, 3H), 5.363-4.532 (br, R-CO-N(-CH₂-CH₂-CH₂-*NH*Boc)₂, 2H), 3.695 (s, ArC*H₂*-CO- N(-CH₂-CH₂-CH₂-NHBoc)₂, 2H), 3.415-3.022 (m, R-CO-N(-C*H₂*-CH₂-C*H₂*-NHBoc)₂, 8H), 1.662-1.629 (br, R-CO-N((-CH₂-C*H*₂-CH₂-NHBoc)₂, 4H), 1.438-1.421 (d, R-CO-N(-CH₂-CH₂-CH₂-NH-COO-C(C*H₃)₃*)₂, 18H). HRMS (m/z): 472.2714 [(M+Na)⁺] (Observed), 472.2787 [(M+Na)⁺] (Calculated).

### N,N-bis(3-(Boc-amino)propyl) naphthyl ethanamide (5c):

Yield-55%; ¹H-NMR (400 MHz, CDCl₃) δ/ppm: 7.791-7.532 (m, Ar*H*, 3H), 7.515-7.407 (m, Ar*H*, 4H), 5.268-4.484 (br, R-CO-N(-CH₂-CH₂-CH₂-*NH*Boc)₂, 2H), 4.133(s, ArC*H*₂-CO-N(N(-CH₂-CH₂-CH₂-*NH*Boc)₂, 2H), 3.461-3.037 (m, R-CO-N(-*CH*₂-CH₂-C*H₂*-NHBoc)₂, 8H), 1.709-1.670 (br, R-CO-N((-CH₂-C*H*₂-CH₂-NHBoc)₂, 4H), 1.411-1.404 (d, R-CO-N(-CH₂-CH₂-CH₂-NH-COO-C(C*H₃)₃*)₂, 18H). HRMS (m/z): 522.2935 [(M+Na)⁺] (Observed), 522.2944 [(M+Na)⁺] (Calculated).

### N,N-bis(3-(Boc-amino)propyl) biphenyl methanamide (5d):

Yield-70%; ¹H-NMR (400 MHz, CDCl₃) δ/ppm: 7.642-7.355 (m, Ar*H*, 9H), 5.367 and 4.357 (br, R-CO-N(-CH₂-CH₂-CH₂-*NH*Boc)₂, 2H), 3.603-2.994 (br, R-CO-N(-*CH₂*-CH₂-*CH₂*-NHBoc)₂, 8H), 1.806-1.670 (br, R-CO-N((-CH₂-C*H*₂-CH₂-NHBoc)₂, 4H), 1.496-1.357 (br, R-CO-N(-CH₂-CH₂-CH₂-NH-COO-C(C*H₃)₃*)₂, 18H). HRMS (m/z): 534.2934 [(M+Na)⁺] (Observed), 534.2944 [(M+Na)⁺] (Calculated).

### N,N-bis(3-(Boc-amino)propyl) 3,3-diphenyl propanamide (5e):

Yield-74%; ¹H-NMR (400 MHz, CDCl₃) δ/ppm: 7.288-7.284 (ArH, 3H), 7.252-7.154 (m, Ar*H*, 7H), 5.227 (br, -N*H*Boc, 1H), 4.733-4.695 (t, Ar*CH*CH₂-CO-N(-CH₂-CH₂-CH₂-NHBoc)₂, 1H), 4.560 (br, -N*H*Boc, 1H), 3.337-3.055 and 2.783-2.739 (m and q, R-CO-N(-*CH₂*-CH₂-*CH₂*-NHBoc)₂, 8H), 3.036-3.017 (d, ArCH*CH₂*-CO-N(-CH₂-CH₂-CH₂-NHBoc)₂, 2H), 1.636-1.439 (br, R-CO-N(-CH₂-*CH₂*-CH₂-NHBoc)₂, 4H), 1.434 (s, R-CO-N(-CH₂-CH₂-CH₂-NH-COO-C(C*H₃)₃*)₂, 18H). HRMS (m/z): 540.3900 [(M+H)⁺] (Observed), 540.3437 [(M+H)⁺] (Calculated).

### N,N-bis(3-(Boc-amino)propyl) 2'-(Boc-amino), 3'-indolo propanamide (5f):

Yield-69%; %; ¹H-NMR (400 MHz, CDCl₃) δ/ppm: 8.334 (s, -N*H*_{indole}, 1H), 7.717-7.698 (d, Ar*H*, 1H), 7.360-7.340 (d, Ar*H*, 1H), 7.210-7.034 (m, Ar*H*, 3H), 5.331-4.586 (br, ArCH₂C*H*(N*H*Boc)CO-N((-CH₂-CH₂-CH₂-N*H*Boc)₂, 4H), 3.409-2.703 (m, ArC*H₂*CH(NHBoc)CO-N(-C*H₂*-CH₂-*CH₂*-NHBoc)₂, 10H), 1.728- 1.714 (br, R-CO-N(-CH₂-*CH₂*-CH₂-NHBoc)₂, 4H), 1.452-1.434 (d, ArCH₂CH(NH-COO-C(C*H₃)₃*)CO-N(-CH₂-CH₂-CH₂-NH-COO-C(C*H₃)₃*)₂, 27H). HRMS (m/z): 618.3531 [(M+H)⁺] (Observed), 618.3867 [(M+H)⁺] (Calculated).

### General procedure for synthesizing final compounds (Compound 1 to Compound 5):

At first 1 equivalent of **5b-5f** was dissolved in dry DCM. To the intensely stirred solution, 4 equivalents (excess amount) of trifluoroacetic acid (TFA) was added and allowed to stir at RT for 4h. After that reaction, solvent and unused TFA were removed to get the pure product with 100% yield with CF₃COO⁻ counterions. The final compounds can be synthesized with other counterions such as Cl⁻, Br⁻ etc.

### N,N-bis((3-amino)propyl)phenyl ethanamide bis(trifluoroacetate) (Compound 1):

¹H-NMR (400 MHz, DMSO-d₆) δ/ppm: 7.827 (br, R-CO-N(-CH₂-CH₂-CH₂-*NH₃⁺*)*₂,* 6H), 7.327-7.290 (Ar*H*, 2H), 7.248-7.231 (d, Ar*H*, 3H), 3.700 (s, ArC*H₂*-CO-N(-CH₂-CH₂-CH₂-NH₃⁺)₂, 2H), 3.411-3.370 (t, R-CO-N(-CH₂-C*H₂*-CH₂-NH₃⁺)₂, 2H), 3.333-3.297 (R-CON(-CH₂-CH₂-C*H₂*-NH₃⁺)₂, 2H), 2.856-2.820 (t, R-CO-N-(*CH₂*-CH₂-CH₂-NH₃⁺)₂, 2H), 2.731-2.695 (t, R-CO-N-(*CH₂*-CH₂-CH₂-NH₃⁺)₂, 2H), 1.862-1.706 (m, R-CO-N(-CH₂-C*H*₂-CH₂-NH₃⁺)₂, 4H). HRMS (m/z): 250.9104 [(M+H)⁺] (Observed), 250.1919 [(M+H)⁺] (Calculated).

### N,N-bis((3-amino)propyl)naphthyl ethanamide bis(trifluoroacetate) (Compound 2) (not part of the present invention):

¹H-NMR (400 MHz, DMSO-d₆) δ/ppm: 7.913-7.824 (m, R-CO-N(-CH₂-CH₂-CH₂-*NH₃⁺*)*₂* and Ar*H*, 8H), 7.542-7.329 (m, Ar*H*, 5H), 4.176 (s, ArC*H₂*-CO-N(-CH₂-CH₂-CH₂-NH₃⁺)₂, 2H), 3.556-3.517 (t, R-CO-N(-CH₂-C*H₂*-CH₂-NH₃⁺)₂, 2H), 3.373-3.339 (t, R-CO-N(-CH₂-CH₂-C*H₂*-NH₃⁺)₂, 2H), 2.901-2.886 (br, R-CO-N-(*CH₂*-CH₂-CH₂-NH₃⁺)₂, 2H), 2.743-2.729 (br, R-CO-N-(*CH₂*-CH₂-CH₂-NH₃⁺)₂, 2H), 1.987-1.889 (quint, R-CO-N(-CH₂-C*H₂*-CH₂-NH₃⁺)₂, 2H), 1.803-1.767 (quint, R-CO-N(-CH₂-C*H₂*-CH₂-NH₃⁺)₂, 2H). HRMS (m/z): 300.2338 [(M+H)⁺] (Observed), 300.2076 [(M+H)⁺] (Calculated).

### N,N-bis((3-amino)propyl)biphenyl methanamide bis(trifluoroacetate) (Compound 3) (not part of the present invention):

¹H-NMR (400 MHz, DMSO-d₆) δ/ppm: 7.961-7.394 (m, Ar*H* and Ar-CO-N(-CH₂-CH₂-CH₂-*NH₃⁺*)₂, 15H), 3.369-3.318 (br, Ar-CO-N(-CH₂-CH₂-*CH₂*-NH₃⁺)₂, 4H), 2.968-2.664 (br, Ar-CO-N-(*CH₂*-CH₂-CH₂-NH₃⁺)₂, 4H), 1.903-1.735 (br, R-CO-N(-CH₂-*CH₂*-CH₂-NH₃⁺)₂, 4H). HRMS (m/z): 312.2069 [(M+H)⁺] (Observed), 312.2076 [(M+H)⁺] (Calculated).

### N,N-bis((3-amino)propyl)3,3-diphenyl propanamide bis(trifluoroacetate) (Compound 4):

¹H-NMR (400 MHz, DMSO-d₆) δ/ppm: 7.845-7.648 (br, R-CO-N(-CH₂-CH₂-CH₂-N*H₃*⁺)₂, 6H), 7.326-7.140 (m, Ar*H*, 10H), 4.555-4.517 (t, ArC*H*CH₂-CO-N((-CH₂-CH₂-CH₂-NH₃⁺)₂, 1H), 3.384-3.346 (t, R-CO-N(-CH₂-C*H₂*-CH₂-NH₃⁺)₂, 2H), 3.235-3.202 (t, R-CO-N(-CH₂-CH₂-C*H₂*-NH₃⁺)₂, 2H), 3.119-3.100 (d, ArCHC*H₂*-CO-N(-CH₂-CH₂-CH₂-NH₃⁺)₂, 2H), 2.972-2.816 (m, R-CO-N(-C*H₂*-CH₂*-*CH₂-NH₃⁺)₂, 4H), 1.808-1.754 (quint, R-CO-N(-CH₂-C*H₂*-CH₂-NH₃⁺)₂, 2H), 1.629-1.578 (quint, R-CO-N(-CH₂-*CH₂*-CH₂-NH₃⁺)₂, 2H). HRMS (m/z): 340.2379 [(M+H)⁺] (Observed), 340.2389 [(M+H)⁺] (Calculated).

### N,N-bis((3-amino)propyl) 2'-(Boc-amino), 3'-indolo propanamide tris(trifluoroacetate) (Compound 5) (not part of the present invention): :

¹H-NMR (400 MHz, DMSO-d₆) δ/ppm: 11.071 (s, -N*H*_{indole}, 1H), 8.232-7.802 (br, ArCH₂CH(N*H₃*⁺)CO-N(-CH₂-CH₂-CH₂-N*H₃*⁺)₂, 9H), 7.524-7.504 (d, ArH, 1H), 7.396-6.993 (m, ArH, 4H), 4.446-4.434 (br, ArCH₂C*H*(NH₃⁺)CO-N(-CH₂-CH₂-CH₂-*N*H₃⁺)₂, 1H), 3.395-3.325 (m, ArC*H₂*CH(NH₃⁺)CO-N(-CH₂-CH₂-CH₂-*N*H₃⁺)₂, 2H), 3.168-2.688 (m, R-CO-N(-C*H₂*-CH₂-C*H₂*-NH₃⁺)₂, 8H), 1.663 (br, R-CO-N(-CH₂-*CH₂*-CH₂-NH₃⁺)₂, 4H). HRMS (m/z): 318.2097 [(M+H)⁺] (Observed), 318.2294 [(M+H)⁺] (Calculated).

### Example 2: Synthesis of N-(Adamantan-2-yl)-2-[N',N'-{bis-(3-amino) propyl} amino] ethanamide tris(trifluoroacetate) (Compound 6): (not part of the present invention)

SMAs where R₁ = R₂ = Hydrogen and R₃ = Cyclic aliphatic radical (adamantyl group) were synthesized from *N¹*-Boc-*N³*-[3- (Boc amino) propyl] propane-1,3-diamine (**5a**) and *N-*(Adamantan-2-yl)-2-bromoethanamide (**2a**), through simple nucleophilic substitution reaction followed by deprotection using trifluoroacetic acid to give the final compounds with CF₃COO⁻as counterions. The final compounds can be synthesized with other counterions such as Cl⁻, Br⁻etc.

### Example 2.1: Synthesis of N-(adamantan-2-yl)-2-bromoethanamide (2a):

About 500 mg of 2-Adamantyl amine hydrochloride (1 equivalent, 2.66 mmol) was dissolved in 12 mL DCM and 552 mg of K₂CO₃ (1.5 equivalents, 3.99 mmol) dissolved in 10 mL of water was added to it at 5⁰C. 806.5 mg of Bromoacetyl bromide (1.5 equivalents, 3.99 mmol) was dissolved in 8 mL DCM and slowly added to the reaction mixture using a dropper funnel. The reaction was kept at 5⁰C for 30 minutes, until the addition of bromoacetyl bromide and then at room temperature for 12h. After 12h, the reaction was stopped, DCM layer collected, worked up with water 3 times and finally evaporated to get the desired pure product with 85% yield. ¹H-NMR (400 MHz, CDCl₃) δ/ppm: 6.914 (br, Ad-N*H*-CO-CH₂Br, 1H), 4.046-4.025 (t, Ad*H*, 1H), 3.912 (s, Ad-NH-CO-*CH₂*Br, 2H), 1.944-1.612 (m, AdH, 14H).

### N-(Adamantan-2-yl)-2-[N',N'-{bis-(3-Boc-amino) propyl} amino] ethanamide (6a):

300 mg of *N¹*-Boc-*N³*-(3-(Boc-amino)-propyl) propane-1,3-diamine, **5a** (1 equivalent, 0.905 mmol) was dissolved in DMF. 187.6 mg of K₂CO₃ (1.5 equivalents, 1.36 mmol) was added to the reaction mixture. Then, 246.33 mg of *N*-(adamantan-2-yl)-2-bromoethanamide, **2a** (1 equivalent, 0.905 mmol), dissolved in DMF was added to the reaction mixture. The reaction was stirred at room temperature for 24 hours. The reaction mixture was then dissolved in ethyl acetate and work-up was performed with ice-cold water (3 times) to remove the DMF. The ethyl acetate layer was collected and the solvent evaporated to get the pure product with 80% yield. ¹H-NMR (400 MHz, CDCl₃) δ/ppm: 4.747 (br, Ad-NH-CO-CH₂-N(-CH₂-CH₂-CH₂-N*H*Boc)₂, 2H), 4.126-4.040 (m, Ad*H*, 1H), 3.178-3.087 (br, Ad-NH-CO-*CH₂*-N(-CH₂-CH₂-*CH₂*-NHBoc)₂, 6H), 2.560 (br, Ad-NH-CO-CH₂-N(-*CH₂*-CH₂-CH₂-NHBoc)₂, 4H), 1.903-1.664(br, Ad*H* and Ad-NH-CO-CH₂-N(-CH₂-C*H₂*-CH₂-NHBoc)₂, 17H), 1.432 (s, Ad-NH-CO-CH₂-N(-CH₂-CH₂-CH₂-NH-COO-C(C*H₃)*₃)₂, 18H). HRMS (m/z): 523.3971 [(M+H)⁺] (Observed), 523.3859 [(M+H)⁺] (Calculated).

### N-(Adamantan-2-yl)-2-[N',N'-{bis-(3-amino) propyl} amino] ethanamide tris(trifluoroacetate) (Compound 6):

At first 380 mg (1 equivalent, 0.73 mmol) of **6a** was dissolved in dry DCM. To the intensely stirred solution 4 equivalents (excess amount) of trifluoroacetic acid (TFA) was added and allowed to stir at RT for 4-6 h. After that reaction, solvent and unused TFA was removed to get the pure product with 100% yield with CF₃COO- counterions. ¹H-NMR (400 MHz, DMSO-d₆) δ/ppm: 8.391 (br, AdN*H*COCH₂-NH⁺(-CH₂-CH₂-CH₂-NH₃⁺)₂, 1H), 7.937 (br, AdNHCOCH₂-NH⁺(-CH₂-CH₂-CH₂-*NH₃*⁺)₂, 6H), 3.922-3.905 (br, Ad*H*, 1H), 2.990-2.860 (br, AdNHCOC*H₂*-NH⁺(-CH₂-CH₂-C*H₂*-NH₃⁺)₂, 6H), 1.959-1.704 (m, Ad*H* and AdNHCOCH₂-NH⁺(-C*H₂*-CH₂-CH₂-NH₃⁺)₂, 18H), 1.545-1.235 (m, AdNHCOCH₂-NH⁺(-CH₂-C*H₂*-CH₂-NH₃⁺)₂, 4H). HRMS (m/z): 323.2791 [(M+H)⁺] (Observed), 323.2811 [(M+H)⁺] (Calculated).

### Example 3: Synthesis of N,N-bis-[(6-(^{L}Lys)amido) propyl] naphthyl ethanamide tetrakis(trifluoroacetate) (Compound 7): (not part of the present invention)

SMAs where R₁ = R₂ = ^{L}Lysine and R₃ = Aromatic radicals were synthesized from *N¹*-(Boc-^{L}Lys)-*N³*-[{3-(Boc-^{L}Lys)amino} propyl] propane-1,3-diamine, **7a** and different aromatic radical bearing carboxylic acids or amino acids, through simple amide coupling reaction using O-benzotriazole-*N,N,N',N'*-tetramethyl-uronium-hexafluorophosphate (HBTU) as coupling agent followed by deprotection using trifluoroacetic acid to give the final compounds with CF₃COO⁻ as counterions. The final compounds can be synthesized with other counterions such as Cl⁻, Br⁻ etc.

### Synthesis of N¹-(Boc-^{L}Lys)-N³-[{3-(Boc-^{L}Lys)amido} propyl]propane-1,3-diamine (7a):

About 3.17 g (2.4 equivalents, 9.14 mmol) of *N, N*'-DiBoc-L-Lysine was dissolved in about 25 mL of dry DCM at 0°C. In the reaction mixture, about 3.32 mL (5 equivalents, 19.05 mmol) of DIPEA was added followed by about 3.47 g (2.4 equivalents, 9.14 mmol) of HBTU. Now about 8 mL of DMF was added to the reaction mixture. After 10 minutes, about 500 mg (1 equivalent, 3.81 mmol) of norspermidine, dissolved in 10 mL of DCM was added dropwise to the reaction mixture. The reaction mixture was allowed to stir for 48 h at 0°C. Then the reaction solvent was evaporated and the residue was diluted in ethyl acetate. Thereafter work-up was done with 1N HCl (50 mL, 3 times) followed by saturated Na₂CO₃ solution (50 mL, 3 times). The crude product was collected in the ethyl acetate layer. Finally, column chromatography was done using different ratios of chloroform and methanol solution, to isolate the product with 42% yield. ¹H-NMR (400 MHz, CDCl₃) δ/ppm: 7.337 (br, NH(-(CH₂)₃-N*H-*CO-CH(NHBoc)-(CH₂)₄-NHBoc)₂, 2H), 5.566 (br, NH(-(CH₂)₃-NH-CO-CH(N*H*Boc)-(CH₂)₄-NHBoc)₂, 2H), 4.808 (br, NH(-(CH₂)₃-NH-CO-CH(NHBoc)-(CH₂)₄-N*H*Boc)₂, 2H), 4.053 (br, NH(-(CH₂)₃-NH-CO-C*H*(NHBoc)-(CH₂)₄-NHBoc)₂, 2H), 3.475-3.323 and 3.102-3.088 (br, NH(-(CH₂)₂-C*H₂*-NH-CO-CH(NHBoc)-(CH₂)₃-C*H₂*-NHBoc)₂, 8H), 2.745 (br, NH(-C*H₂*-(CH₂)₂-NH-CO-CH(NHBoc)-(CH₂)₄-NHBoc)₂, 4H), 2.349 and 1.802-1.788 (br, NH(-CH₂-C*H₂*-CH₂-NH-CO-CH(NHBoc)-C*H₂*-(CH₂)₃-NHBoc)₂, 8H), 1.666-1.580 (m, NH(-CH₂-CH₂-CH₂-NH-CO-CH(NHBoc)-(CH₂)₂-C*H₂*-CH₂-NHBoc)₂, 4H), 1.422 (br, NH(-(CH₂)₃-NH-CO-CH(NH-COO-C(C*H₃)₃*)-CH₂-C*H₂*-CH₂-CH₂-NH-COO-C(C*H₃)₃*)₂, 40H). HRMS (m/z): 788.5462 [(M+H)⁺] (Observed), 788.5497 [(M+H)⁺] (Calculated).

### N,N-bis-[{3-(Boc-^{L}Lys)amido}propyl]naphthyl ethanamide (7b):

113.4 mg (1.2 equivalents, 0.609 mmol) of Naphthyl acetic acid was dissolved in dry DCM (4 mL) at 0 °C. In the reaction mixture, 265 µL (3 equivalents, 1.52 mmol) of *N,N-*diisopropylethylamine (DIPEA) was added followed by 231 mg (1.2 equivalents, 0.609 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 400 mg (1 equivalent, 0.51 mmol) of *N¹*-(Boc-^{L}Lys)-*N*³-[{3-(Boc-^{L}Lys) amido} propyl] propane-1,3-diamine, **7a** dissolved in dry DCM (4 mL) was added dropwise to the reaction mixture. The reaction mixture was brought to room temperature and allowed to stir for 24 h. Solvent was evaporated, and residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as an eluent to afford compound **7b** with 51% yield. ¹H-NMR (400 MHz, CDCl₃) δ/ppm: 7.930-7.909 (d, ArH, 1H), 7.866-7.847 (d, Ar*H*, 1H), 7.844-7.775 (d, Ar*H*, 1H), 7.536-7.482 (quint, Ar*H*, 2H), 7.465-7.407 (t, Ar*H*, 1H), 7.387-7.321 (d, Ar*H*, 1H), 7.172 (br, R-CO-N(-(CH₂)₃-N*H*-CO-CH(NHBoc)-(CH₂)₄-NHBoc)₂, 2H), 5.352 (br, R-CO-N(-(CH₂)₃-NH-CO-CH(N*H*Boc)-(CH₂)₄-NHBoc)₂, 2H), 5.257-5.240 (br, R-CO-N(-(CH₂)₃-NH-CO-CH(NHBoc)-(CH₂)₄-N*H*Boc)₂, 2H), 4.667 (br, R-CO-N((-(CH₂)₃-NH-CO-C*H*(NHBoc)-(CH₂)₄-NHBoc)₂, 2H), 4.120 (s, ArCH₂-CO-N(-(CH₂)₃-NH-CO-CH(NHBoc)-(CH₂)₃-C*H₂*-NHBoc)₂, 4H), 3.473-3.362 (br, R-CO-N(-(CH₂)₂-C*H₂*-NH-CO-CH(NHBoc)-(CH₂)₄-NHBoc)₂, 4H), 3.243 (br, ArC*H₂*-CO-N(-(CH₂)₃-NH-CO-CH(NHBoc)-(CH₂)₃-CH₂-NHBoc)₂, 2H), 3.079-3.047 (t, R-CO-N(-C*H₂*-(CH₂)₂-NH-CO-CH(NHBoc)-(CH₂)₃-CH₂-NHBoc)₂, 4H), 1.772 (br, R-CO-N(-CH₂-C*H₂*-CH₂-NH-CO-CH(NHBoc)-(CH₂)₃-CH₂-NHBoc)₂, 4H), 1.689 (br, R-CO-N(-(CH₂)₃-NH-CO-CH(NHBoc)-C*H₂*-(CH₂)₃-NHBoc)₂, 4H), 1.595 (br, R-CO-N(-(CH₂)₃-NH-CO-CH(NHBoc)-(CH₂)₂-C*H₂*-CH₂-NHBoc)₂, 4H), 1.430-1.396 (d, R-CO-N(-(CH₂)₃-NH-CO-CH(NH-COO-C(C*H₃)₃*)-(CH₂)₄-NH-COO-C(C*H₃)₃*)₂, 36H), 1.254 (br, R-CO-N(-(CH₂)₃-NH-CO-CH(NHBoc)-CH₂-CH₂-C*H₂*-CH₂-CH₂-NHBoc)₂, 4H). HRMS (m/z): 956.5919 [(M+H)⁺] (Observed), 956.6072 [(M+H)⁺] (Calculated).

### N,N-bis-[(6-(^{L}Lys)amido)propyl]naphthyl ethanamide tetrakis(trifluoroacetate) (Compound 7):

At first 250 mg (1 equivalent, 0.26 mmol) of **7b** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 8.581-8.426 (dt, Ar*H*, 2H), 8.164-8.130 (d, Ar*H*, -N*H*_{amide} and -N*H₃⁺,* 6H), 7.947.7.753 (m, Ar*H*, -N*H*_{amide} and -N*H₃⁺,* 9H), 7.531-7.316 (m, Ar*H* and -N*H₃⁺,* 4H), 4.128 (s, ArC*H₂*CO-N(-(CH₂)₃-NH-CO-CH(NH₃⁺)-(CH₂)₃-CH₂-NH₃⁺)₂, 2H), 3.699-3.686 (br, R-CO-N(-(CH₂)₃-NH-CO-C*H*(NH₃⁺)-(CH₂)₃-CH₂-NH₃⁺)₂, 2H), 3.461-3.309 (br, R-CO-N(-(CH₂)₃-NH-CO-CH(NH₃⁺)-(CH₂)₃-C*H₂*-NH₃⁺)₂, 4H), 3.203-3.109 and 2.744-2.694 (br, R-CON(-C*H₂*-CH₂-C*H₂*-NH-CO-CH(NH₃⁺)-(CH₂)₃-CH₂-NH₃⁺)₂, 8H), 1.821-1.656 (m, R-CO-N(-CH₂-CH₂-CH₂-NH-CO-CH(NH₃⁺)-C*H₂*-CH₂-C*H₂*-CH₂-NH₃⁺)₂, 8H), 1.533-1.457 (m, R-CON(-CH₂-CH₂-C*H₂*-NH-CO-CH(NH₃')-CH₂-CH₂-CH₂-CH₂-NH₃⁺)₂, 8H), 1.332-1.235 (m, R-CO-N(-CH₂-CH₂-CH₂-NH-CO-CH(NH₃⁺)-CH₂-C*H₂*-CH₂-CH₂-NH₃⁺)₂, 4H). HRMS (m/z): 556.3870 [(M+H)⁺] (Observed), 556.3975 [(M+H)⁺] (Calculated).

### Example 4: Synthesis of N,N-bis-[(6-(^{L}Lys)amido) propyl] 3,3-diphenyl propanamide tetrakis(trifluoroacetate) (Compound 8):_(not part of the present invention) N,N-bis-[{3-(Boc-^{L}Lys)amido}propyl] 3,3-diphenylpropanamide (7c):

275.6 mg (1.2 equivalents, 1.22 mmol) of 3,3-diphenyl propionic acid was dissolved in dry DCM (4 mL) at 0 °C. In the reaction mixture, 530.5 µL (3 equivalents, 3.04 mmol) of *N,N-*diisopropylethylamine (DIPEA) was added followed by 462 mg (1.2 equivalents, 1.22 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 800 mg (1 equivalent, 1.01 mmol) of *N¹*-(Boc-^{L}Lys)-*N³*-[{3-(Boc-^{L}Lys) amido} propyl] propane-1,3-diamine, **7a** dissolved in dry DCM (4 mL) was added dropwise to the reaction mixture. The reaction mixture was brought to room temperature and allowed to stir for 24 h. The solvent was evaporated, and the residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as an eluent to afford compound **7c** with 57% yield. ¹H-NMR (400 MHz, CDCl₃) δ/ppm: 7.241-7.147 (m, Ar*H*, 10H), 5.345-5.235 (br, -N*H*_{amide}, 3H), 4.719-4.682 (t, ArC*H*CH₂CO-N(-(CH₂)₃-NH-CO-C*H*(NHBoc)-(CH₂)₃-CH₂-NHBoc)₂, 3H), 4.146-4.092 (q, -N*H*_{amide}, 3H), 3.388-2.788 (m, ArCHC*H₂*CO-N(-C*H₂*-C*H₂*-C*H₂*-NH-CO-CH(NHBoc)-(CH₂)₃-C*H₂*-NHBoc)₂, 18H), 1.691-1.590 (br, R-CO-N(-(CH₂)₃-NH-CO-CH(NHBoc)-C*H₂*-CH₂-C*H₂*-CH₂-NHBoc)₂, 8H), 1.432-1.422 (d, R-CO-N(-(CH₂)₃-NH-CO-CH(NH-COO-C(C*H₃)₃*-CH₂-CH₂-CH₂-CH₂-NH-COO-C(C*H₃)₃*)₂, 36H), 1.419-1.338 (br, R-CO-N(-(CH₂)₃-NH-CO-CH(NHBoc)-CH₂-C*H₂*-CH₂-CH₂-NHBoc)₂, 4H). HRMS (m/z): 996.6348 [(M+H)⁺] (Observed), 996.6385 [(M+H)⁺] (Calculated).

At first 490 mg (1 equivalent, 0.49 mmol) of **7c** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 8.558 (t, -N*H*_{amide}, 1H), 8.318 (t, -N*H*_{amide}, 1H), 8.172-7.801 (br, R-CO-N(-CH₂-CH₂-CH₂-NH-CO-CH(N*H₃*⁺)-CH₂-CH₂-CH₂-CH₂-N*H₃*⁺)₂, 12H), 7.308-7.130 (m, Ar*H*, 10H), 4.539-4.502 (t, ArC*H*CH₂-CO-N(-CH₂-CH₂-CH₂-NH-CO-CH(NH₃⁺)-CH₂-CH₂-CH₂-CH₂-NH₃⁺)₂, 1H), 3.683-3.670 (br, ArCHC*H*₂-CO-N(-CH₂-CH₂-CH₂-NH-CO-C*H*(NH₃⁺)-CH₂-CH₂-CH₂-CH₂-NH₃⁺)₂, 3H), 3.330-2.730 (m, R-CO-N(-C*H*₂-CH₂-C*H*₂-NH-CO-CH(NH₃⁺)-C*H*₂-CH₂-CH₂-C*H*₂-NH₃⁺)₂, 12H), 1.700-1.235 (m, R-CO-N(-CH₂-C*H₂*-CH₂-NH-CO-CH(NH₃⁺)-CH₂-C*H₂*-C*H₂*-CH₂-NH₃⁺)₂, 16H). HRMS (m/z): 596.4248 [(M+H)⁺] (Observed), 596.4288 [(M+H)⁺] (Calculated).

### Example 5: Synthesis of N-(adamantan-2-yl)-2-[N',N'-{bis-(3-^{L}Lys) amido)propyl} amino] ethanamide pentakis(trifluoroacetate) (Compound 9): (not part of the present invention)

SMAs where R₁ = R₂ = Lysine and R₃ = Cyclic aliphatic radical (adamantyl group) were synthesized from *N¹*-(Boc-^{L}Lys)-*N*³-[{3-(Boc-^{L}Lys)amido} propyl] propane-1,3-diamine (**7a,** in Example 7.1) and *N*-adamantyl-2-bromoethanamide (**2a,** in Example 2.1), through simple nucleophilic substitution reaction followed by deprotection using trifluoroacetic acid with CF-₃COO⁻ counterions. The final compounds can be synthesized with other counterions such as Cl⁻, Br⁻ etc.

### N-(adamantan-2-yl)-2-[N',N'-{bis-(3-(Boc-^{L}Lys)amido)propyl}amino] ethanamide (8a):

390 mg of *N¹*-(Boc-^{L}Lys)-*N³*-[{3-(boc-^{L}Lys)amido}propyl] propane-1,6-diamine, **7a** (1 equivalent, 0.77 mmol) in DMF. 154.6 mg of K₂CO₃ (1.5 equivalents, 1.12 mmol) was added to the reaction mixture. Then, 203 mg of *N*-(adamantan-2-yl)-2-bromoethanamide (1 equivalent, 0.77 mmol), dissolved in DMF was added to the reaction mixture. The reaction was stopped after 24 hours. The reaction mixture was then dissolved in ethyl acetate and work-up was performed with ice-cold water (3 times) to remove the DMF. The ethyl acetate layer was collected and the solvent evaporated to get the pure product with 40% yield. ¹H-NMR (400 MHz, CDCl₃) δ/ppm: 6.902 (br, AdN*H*-CO-CH₂-N(-CH₂-CH₂-CH₂-NH-CO-CH(NHBoc)-CH₂-CH₂-CH₂-CH₂-NHBoc)₂, 1H), 5.590 (br, AdNH-CO-CH₂-N(-CH₂-CH₂-CH₂-N*H*-CO-CH(NHBoc)-CH₂-CH₂-CH₂-CH₂-NHBoc)₂, 2H), 4.786 (br, AdNH-CO-CH₂-N(-CH₂-CH₂-CH₂-NH-CO-CH(N*H*Boc)-CH₂-CH₂-CH₂-CH₂-NHBoc)₂, 2H), 4.123-4.027 (m, Ad*H* and AdNH-CO-CH₂-N(-CH₂-CH₂-CH₂-NH-CO-C*H*(NHBoc)-CH₂-CH₂-CH₂-CH₂-NHBoc)₂, 3H), 3.461 (br, AdNH-CO-CH₂-N(-CH₂-CH₂-CH₂-NH-CO-CH(NHBoc)-CH₂-CH₂-CH₂-CH₂-NHBoc)₂, 2H), 3.208-2.519 (br, AdNH-CO-C*H₂*-N(-CH₂-CH₂-C*H₂*-NH-CO-CH(NHBoc)-CH₂-CH₂-CH₂-C*H₂*-NHBoc)₂, 10H), 2.039-1.479 (br, Ad*H* and AdNH-CO-CH₂-N(-C*H₂*-C*H₂*-CH₂-NH-CO-CH(NHBoc)-C*H₂*-C*H₂*-C*H₂*-CH₂-NHBoc)₂, 34H), 1.429-1.422 (d, AdNH-CO-CH₂-N(-CH₂-CH₂-CH₂-NH-CO-CH(NH-COO-C(C*H₃)₃*)-CH₂-CH₂-CH₂-CH₂-NH-COO-C(C*H3)₃*)₂, 36H). HRMS (m/z): 979.6745 [(M+H)⁺] (Observed), 979.6807 [(M+H)⁺] (Calculated).

### N-(adamantan-2-yl)-2-[N',N'-{bis-(3-^{L}Lys)amido)propyl}amino] ethanamide pentakis(trifluoroacetate)(Compound 9):

280 mg (1 equivalent, 0.28 mmol) of **8a** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 9.688 (br, AdNH-CO-CH₂-N*H*⁺(-CH₂-CH₂-CH₂-NH-CO-CH(NH₃⁺)-CH₂-CH₂-CH₂-CH₂-NH₃⁺)₂, 1H), 8.673 (br, AdNH-CO-CH₂-NH⁺(-CH₂-CH₂-CH₂-N*H*-CO-CH(NH₃⁺)-CH₂-CH₂-CH₂-CH₂-NH₃⁺)₂, 2H), 8.482 (br, AdN*H*-CO-CH₂-NH⁺(-CH₂-CH₂-CH₂-NH-CO-CH(NH₃⁺)-CH₂-CH₂-CH₂-CH₂-NH₃⁺)₂, 1H), 8.207 (bs, AdNH-CO-CH₂-NH⁺(-CH₂-CH₂-CH₂-NH-CO-CH(N*H₃*⁺)-CH₂-CH₂-CH₂-CH₂-N*H₃*⁺)₂, 6H), 7.836 (bs, AdNH-CO-CH₂-NH⁺(-CH₂-CH₂-CH₂-NH-CO-CH(N*H*₃⁺)-CH₂-CH₂-CH₂-CH₂-N*H*₃⁺)₂, 6H), 3.915-3.864 (t, AdH and AdNH-CO-CH₂-NH⁺(-CH₂-CH₂-CH₂-NH-CO-C*H*(NH₃⁺)-CH₂-CH₂-CH₂-CH₂-NH₃⁺)₂, 3H), 3.820 (s, AdNH-CO-C*H₂*-NH⁺(-CH₂-CH₂-CH₂-NH-CO-CH(NH₃⁺)-CH₂-CH₂-CH₂-CH₂-NH₃⁺)₂, 2H), 3.167-3.142 (br, AdNH-CO-C*H₂*-NH⁺(-CH₂-CH₂-CH₂-NH-CO-CH(NH₃⁺)-CH₂-CH₂-CH₂-C*H₂*-NH₃⁺)₂, 8H), 2.758 (br, AdNH-CO-CH₂-NH⁺(-CH₂-CH₂-C*H₂*-NH-CO-CH(NH₃⁺)-CH₂-CH₂-CH₂-CH₂-NH₃⁺)₂, 4H), 1.823-1.518 (br, Ad*H* and AdNH-CO-CH₂-NH⁺(-CH₂-C*H₂*-CH₂-NH-CO-CH(NH₃⁺)-C*H₂*-C*H₂*-C*H₂*-CH₂-NH₃⁺)₂, 30H). HRMS (m/z): 579.4681 [(M+H)⁺] (Observed), 579.4710 [(M+H)⁺] (Calculated).

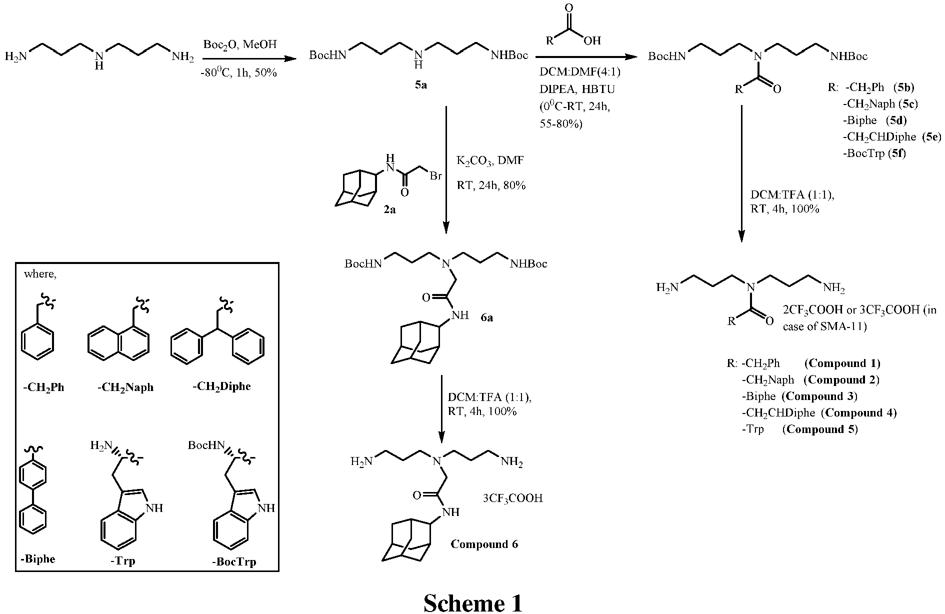

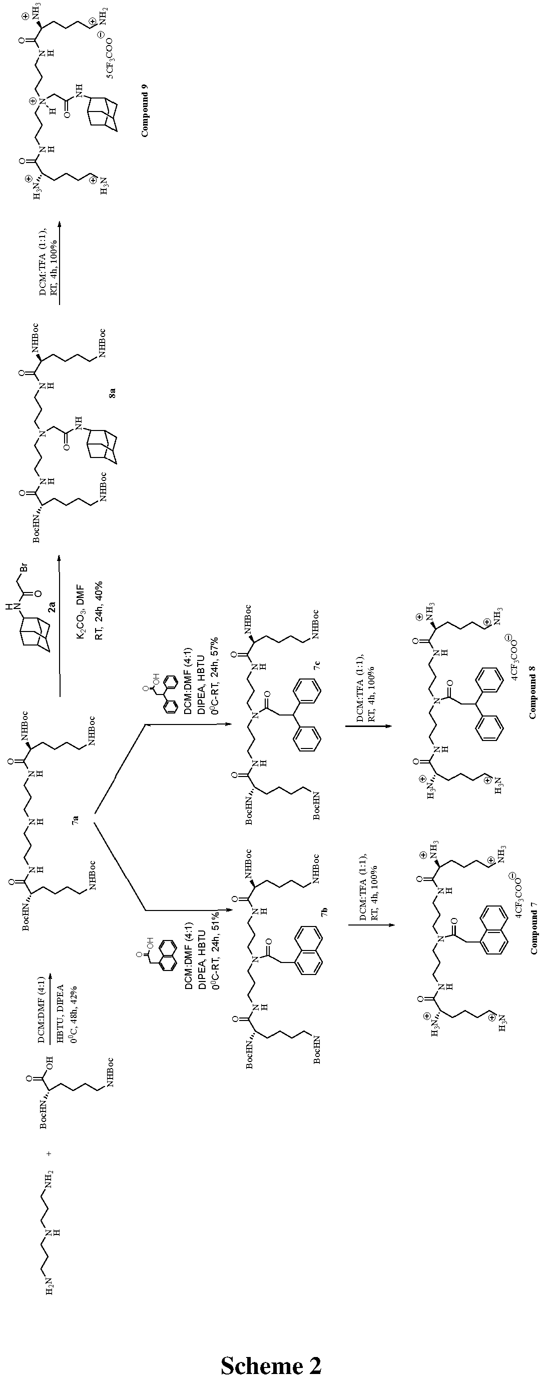

### Example 6: Synthesis of N,N-bis-[(6-amino) hexyl] naphthyl ethanamide bistrifluoroacetate (Compound 10): (not part of the present invention)

### Synthesis of N¹-Boc-N⁶-(6-(Boc-amino)-hexyl) hexane-1,6-diamine (1a):

About 2 g (1 equivalent, 9.29 mmol) of Bis(hexamethylene) triamine was dissolved in about 20 mL of methanol and the solution was kept at -80°C. Then, 3.04 g (1.5 equivalents, 3.93 mmol) of di-tert-butyl dicarbonate (Boc₂O) was dissolved in MeOH (30 mL) and added to the reaction mixture dropwise. The reaction was continued for 1h at -80 °C. Then the reaction mixture was allowed to come to room temperature. MeOH was removed under reduced pressure and purification was done through column chromatography on silica gel (60-120 mesh) using methanol and chloroform as an eluent to afford the product with 68% yield. ¹H-NMR (400 MHz, CDCl₃) δ/ppm: 4.541 (s, NH(-(CH₂)₆-*NH*Boc)₂, 2H), 3.112-3.065 (q, NH(-(CH₂)s-*CH*₂-NHBoc)₂, 4H), 2.602-2.565 (t, NH(-C*H₂*-(CH₂)s-NHBoc)₂, 4H), 1.930 (s, *NH*(-(CH₂)₆-NHBoc)₂, 1H), 1.507-1.296 (m, NH(-CH₂-*(CH₂)₄*-CH₂-NH-COO-C*(CH₃)₃*)₂, 34H). HRMS (m/z): 416.3419 [(M+H) ⁺] (Observed), 416.3488 [(M+H) ⁺] (Calculated).

### N,N-bis-[(6-Boc-amino) hexyl] naphthyl ethanamide (1b):

268.8 mg (1.2 equivalent, 1.44 mmol) of Naphthyl acetic acid was dissolved in dry DCM (4 mL) at 0 °C. In the reaction mixture, 628.6 µL (3 equivalents, 3.609 mmol) of N,N-diisopropylethylamine (DIPEA) was added followed by 547.5 mg (1.2 equivalents, 1.44 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 500 mg (1 equivalent, 1.2 mmol) of *N¹*-Boc-*N⁶*-(6-(boc-amino)-hexyl) hexane-1,6-diamine (**1a**), dissolved in dry DCM (4 mL) was added dropwise to the reaction mixture. The reaction mixture was brought to room temperature and allowed to stir for 24 h. The solvent was evaporated, and residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as eluent to afford compound **1b** with 68% yield. ¹H-NMR (400 MHz, CDCl₃) δ/ppm: 7.541-7.527 (d, Ar*H*, 1H), 7.524-7.520 (d, Ar*H*, 1H), 7.507-7.503 (d, Ar*H*, 1H), 7.500-7.329 (m, ArH, 4H), 4.417 (s, R-CO-N(-(CH₂)₆-*NH*Boc)₂, 2H), 4.115 (s, Ar*CH₂*CO- N(-(CH₂)₆-NHBoc)₂, 2H), 3.363-3.037 (m, R-CO-N(-*CH₂*-(CH₂)₄-*CH₂*-NHBoc)₂, 8H), 1.743 (br, R-CO-N(-CH₂-*CH₂*-(CH₂)₄-NHBoc)₂, 4H), 1.593-1.253 (m, R-CO-N(-CH₂-(CH₂)₃-*CH₂-CH₂*-NH-COO-C(C*H₃)₃*)₂*,* 22H), 1.187-1.171 (m, R-CO-N(-CH₂-CH₂-*(CH₂)₂*-CH₂-CH₂-NHBoc)₂, 8H). HRMS (m/z): 584.4106 [(M+H) ⁺] (Observed), 584.40635 [(M+H) ⁺] (Calculated).

### N,N-bis-[(6-amino) hexyl] naphthyl ethanamide bistrifluoroacetate (Compound 10):

480 mg (1 equivalent, 0.822 mmol) of **1b** was dissolved in dry DCM. To the intensely stirred solution 4 equivalents (excess amount) of trifluoroacetic acid (TFA) was added and allowed to stir at RT for 4-6 h. After that reaction, solvent and unused TFA was removed to get the pure product with 100% yield with CF₃COO⁻ counterions. ¹H-NMR (400 MHz, DMSO-d₆) δ/ppm: 7.921-7.707 (m, Ar*H* and R-CO-N-((CH₂)₆-N*H₃*⁺)₂), 9H), 7.538-7.426 (m, Ar*H*, 3H), 7.343-7.326 (d, Ar*H*, 1H), 4.117 (s, Ar*CH₂*CO- N(-(CH₂)₆-NH₃⁺)₂, 2H), 3.276-3.240 (t, R-CO-N((CH₂)₅-*CH₂*-NH₃⁺)₂, 4H), 2.772-2.722 (q, R-CO-N-(*CH*₂-(CH₂)₅-NH₃⁺)₂, 4H), 1.536-1.440 (m, R-CO-N-(CH₂-*CH*₂-(CH₂)₂-*CH₂*-CH₂-NH₃⁺)₂, 8H), 1.335-1.220 (m, R-CO-N-(CH₂-CH₂-*(CH₂)₂*-CH₂-CH₂-NH₃⁺)₂, 8H). HRMS (m/z): 384.3040 [(M+H) ⁺] (Observed), 384.3015 [(M+H) ⁺] (Calculated).

### Example 7: Synthesis of N,N-bis-[(6-amino) hexyl] 3,3-diphenyl propanamide bistrifluoroacetate (Compound 11): (not part of the present invention)

### N,N-bis-[(6-Boc-amino) hexyl] 3,3-diphenyl propanamide (1c):

326.6 mg (1.2 equivalents, 1.44 mmol) of 3,3-Diphenyl propionic acid was dissolved in dry DCM (4 mL) at 0 °C. In the reaction mixture, 628.65 µL (3 equivalents, 3.609 mmol) of *N,N*-diisopropylethylamine (DIPEA) was added followed by 547.48 mg (1.2 equivalents, 1.44 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 500 mg (1 equivalent, 1.203 mmol) of *N¹-Boc-N⁶*-(6-(boc-amino)-hexyl) hexane-1,6-diamine (**1a**), dissolved in dry DCM (4 mL) was added dropwise to the reaction mixture. The reaction mixture was brought to room temperature and allowed to stir for 24 h. Solvent was evaporated, and residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as eluent to afford compounds **1c** with 68% yield. ¹H-NMR (400 MHz, CDCl₃) δ/ppm: 7.282 (s, Ar*H*, 1H), 7.245-7.147 (m, Ar*H*, 9H), 4.741-4.704 (t, Ar*CH*CH₂CO-N(-(CH₂)₆-NHBoc)₂, 1H), 4.522 (br, R-CO-N(-(CH₂)₆-N*H*Boc)₂, 2H), 3.227-3.059 (m, R-CO-N(*CH₂*-(CH₂)₄-*CH₂*-NHBoc)₂, 8H), 3.003-3.984 (d, ArCH*CH₂*CO-N(-(CH₂)₆-NHBoc)₂, 2H), 1.442 (s, R-CO-N(-(CH₂)₆-NH-COO-C*(CH₃)₃*)₂, 18H), 1.406-1.101 (m, R-CO-N(-CH₂-*(CH₂*)₄-CH₂-NHBoc)₂,16H). HRMS (m/z): 624.4347 [(M+H) ⁺] (Observed), 624.4376 [(M+H) ⁺] (Calculated).

510 mg (1 equivalent, 0.817 mmol) of **1c** was dissolved in dry DCM. To the intensely stirred solution 4 equivalents (excess amount) of trifluoroacetic acid (TFA) was added and allowed to stir at RT for 4-6 h. After that reaction, solvent and unused TFA was removed to get the pure product with 100% yield with CF₃COO⁻ counterions. ¹H-NMR (400 MHz, DMSO-d₆) δ/ppm: 7.735 (bs, R-CO-N-((CH₂)₆-N*H₃*⁺)₂, 6H), 7.296-7.097 (m, Ar*H*, 10H), 4.541-4.504 (t, Ar*CH*CH₂CO-N(-(CH₂)₆-NH₃⁺)₂, 1H), 3.241-3.109 (dt, R-CO-N(CH₂-(CH₂)₄-*CH₂*-NH₃⁺)₂, 4H), 3.036-3.018 (d, ArCH*CH₂*CO-N(-(CH₂)₆-NH₃⁺)₂, 2H), 2.788-2.701 (m, R-CO-N(*CH₂*-(CH₂)₄-CH₂-NH₃⁺)₂, 4H), 1.532-1.077 (m, R-CO-N(CH₂-*(CH₂)₄*-CH₂-NH₃⁺)₂, 16H). HRMS (m/z): 424.3299 [(M+H) ⁺] (Observed), 424.3328 [(M+H)⁺] (Calculated).

### Example 8: Synthesis of N-(adamantan-2-yl)-2-[N',N'-{bis-(6-amino) hexyl} amino] ethanamide tristrifluoroacetate (Compound 12): (not part of the present invention)

### Synthesis of N-(adamantan-2-yl)-2-bromoethanamide (2a):

About 500 mg of 2-Adamantyl amine hydrochloride (1 equivalent, 2.66 mmol) was dissolved in 12 mL DCM and 552 mg of K₂CO₃ (1.5 equivalents, 3.99 mmol) dissolved in 10 mL of water was added to it at 5⁰C. 806.5 mg of Bromoacetyl bromide (1.5 equivalents, 3.99 mmol) was dissolved in 8 mL DCM and slowly added to the reaction mixture using a dropper funnel. The reaction was kept at 5⁰C for 30 minutes, till the addition of Bromoacetyl bromide and then at room temperature for 12h. After 12h, the reaction was stopped, DCM layer collected, worked up with water 3 times and finally evaporated to get the desired pure product with 85% yield. ¹H-NMR (400 MHz, CDCl₃) δ/ppm: 6.914 (br, Ad-N*H*-CO-CH₂Br, 1H), 4.046-4.025 (t, Ad*H*, 1H), 3.912 (s, Ad-NH-CO-*CH₂*Br, 2H), 1.944-1.612 (m, Ad*H*, 14H).

### N-(adamantan-2-yl)-2-[N',N'-{bis-(6-Boc-amino) hexyl} amino] ethanamide (2b):

300 mg of *N¹*-Boc-*N⁶*-(6-(Boc-amino)-hexyl) hexane-1,6-diamine, **1a** (1 equivalent, 0.72 mmol) was dissolved in DMF. 149.6 mg of K₂CO₃ (1.5 equivalents, 1.1 mmol) was added to the reaction mixture. Then, 196.5 mg of *N*-(adamantan-2-yl)-2-bromoethanamide, **2a** (1 equivalent, 0.72 mmol), dissolved in DMF was added to the reaction mixture. The reaction was stirred at room temperature for 24 hours. The reaction mixture was then dissolved in ethyl acetate and work-up was performed with ice-cold water (3 times) to remove the DMF. The ethyl acetate layer was collected and the solvent evaporated to get the pure product with 61% yield. ¹H-NMR (400 MHz, CDCl₃) δ/ppm: 8.575 (br,Ad-N*H*-CO-CH₂-N(-(CH₂)₆-NHBoc)₂, 1H), 4.660-4.489 (br, Ad-NH-CO-CH₂-N(-(CH₂)₆-N*H*Boc)₂, 2H), 4.145-4.027 (m, Ad*H*, 1H), 3.103 (br, Ad-NH-CO-*CH*₂-N(-CH₂-(CH₂)₄-*CH₂*-NHBoc)₂, 6H), 2.041-1.594 (m, Ad*H* and -*CH₂*-_{(Boc-aminohexyl)} ,26H), 1.440 (s, Ad-NH-CO-CH₂-N(-(CH₂)₆-NH-COO-C*(CH₃)₃*)₂, 18H), 1.375-1.238 (m, Ad*H* and -*CH₂*-_{(Boc-aminohexyl)} ,8H). HRMS (m/z): 607.4764 [(M+H)⁺] (Observed), 607.4798 [(M+H)⁺] (Calculated).

### N-(adamantan-2-yl)-2-[N',N'-{bis-(6-amino) hexyl} amino] ethanamide tristrifluoroacetate (Compound 12):

270 mg (1 equivalent, 0.445 mmol) of **2b** was dissolved in dry DCM. To the intensely stirred solution 4 equivalents (excess amount) of trifluoroacetic acid (TFA) was added and allowed to stir at RT for 4-6 h. After that reaction, solvent and unused TFA was removed to get the pure product with 100% yield with CF₃COO⁻ counterions. ¹H-NMR (400 MHz, DMSO-d₆) δ/ppm: 8.540-8.457 (br, Ad-N*H*-CO-CH₂-NH⁺(-(CH₂)₆-NH₃⁺)₂, 1H), 7.800 (br, Ad-NH-CO-CH₂-NH⁺(-(CH₂)₆-N*H₃*⁺)₂, 6H), 3.902-3.820 (m, Ad*H* and Ad-NH-CO-*CH₂*-NH⁺(-(CH₂)₆-NH₃⁺)₂, 3H), 3.082-2.669 (br, Ad-NH-CO-CH₂-NH⁺(*-CH₂*-(CH₂)₄-*CH₂*-NH₃⁺)₂, 8H), 1.967-1.235 (m, Ad*H* and *-CH₂*-_{(aminohexyl)}, 30H). HRMS (m/z): 407.3724 [(M+H)⁺] (Observed), 407.3749 [(M+H)⁺] (Calculated).

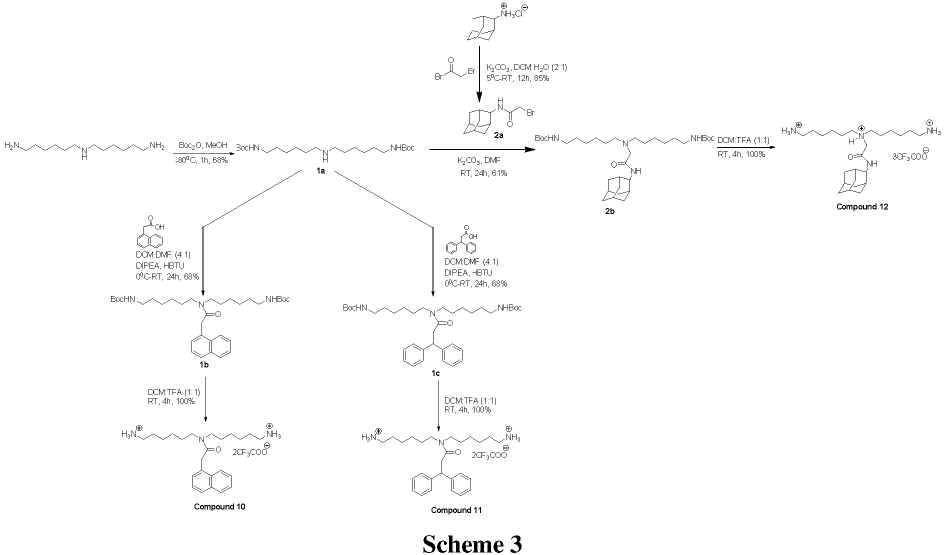

### Example 9: Synthesis of N,N-bis-[(6-(^{L}Lys)amido) hexyl] naphthyl ethanamide tetrakistrifluoroacetate (Compound 13): (not part of the present invention)

### Synthesis of N¹-(Boc-^{L}Lys)-N⁶-[{6-(Boc-^{L}Lys)amido} hexyl] hexane-1,6-diamine (3a):

About 6.4 g (2 equivalents, 18.56 mmol) of N, *N*'-DiBoc-L-Lysine was dissolved in about 25 mL of dry DCM at 0°C. In the reaction mixture, about 6.5 mL (4 equivalents, 37.1 mmol) of DIPEA was added followed by about 7.0 g (2 equivalents, 18.6 mmol) of HBTU. Now about 8 mL of DMF was added to the reaction mixture. After 10 minutes, about 2 g (1 equivalent, 9.3 mmol) of Bis(hexamethylene) triamine, dissolved in 10 mL of DCM was added dropwise to the reaction mixture. The reaction mixture was allowed to stir for 48 h at 0°C. Then reaction solvent was evaporated and the residue was diluted in ethyl acetate. Thereafter work-up was done with 1N HCl (50 mL, 3 times) followed by saturated Na₂CO₃ solution (50 mL, 3 times). The crude product was collected in ethyl acetate layer. Finally, column was done using different ratios of chloroform and methanol solution, to isolate the product with 34% yield. ¹H-NMR (400 MHz, CDCl₃) δ/ppm: 6.489-6.323 (m, NH(-(CH₂)₆-N*H*-CO-CH(NHBoc)-(CH₂)₄-NHBoc)₂, 2H), 5.296 (br, NH(-(CH₂)₆-NH-CO-CH(N*H*Boc)(CH₂)₄-NHBoc)₂, 2H), 4.705 (br, NH(-(CH₂)₆-NH-CO-CH(NHBoc)((CH₂)₄-N*H*Boc)₂, 2H), 4.020 (br, NH(-(CH₂)₆-NH-CO-*CH*(NHBoc)(CH₂)₄-NHBoc)₂, 2H), 3.246-3.075 (m, NH(-(CH₂)₅-*CH₂*-NH-CO-CH(NHBoc)(CH₂)₃-*CH₂*-NHBoc)₂, 8H), 2.729 (br, NH(-*CH₂*-(CH₂)₅₋NH-CO-CH(NHBoc)(CH₂)₄-NHBoc)₂, 4H), 1.836-1.480 (m, NH(-CH₂-*CH₂*-(CH₂)₂-*CH₂*-CH₂-NH-CO-CH(NHBoc)(-*CH₂*-CH₂-*CH₂*-CH₂-NHBoc)₂, 16H), 1.431 (s, NH(-(CH₂)₆-NH-CO-CH(NH-COO-C*(CH₃)₃*)(CH₂)₄-NH-COO-C*(CH₃)₃*)₂, 36H), 1.382-1.347 (m, NH(-CH₂-CH₂-*(CH₂)₂*-CH₂-CH₂-NH-CO-CH(NHBoc)(-CH₂-*CH₂*-CH₂-CH₂-NHBoc)₂, 12H). HRMS (m/z): 872.6286 [(M+H) ⁺] (Observed), 872.6436 [(M+H) ⁺] (Calculated).

### N,N-bis-[{6-(Boc-^{L}Lys)amido}hexyl] naphthyl ethanamide (3b):

41 mg (1.2 equivalents, 0.22 mmol) of Naphthyl acetic acid was dissolved in dry DCM (4 mL) at 0 °C. In the reaction mixture, 96 µL (3 equivalents, 0.55 mmol) of *N,N-*diisopropylethylamine (DIPEA) was added followed by 83.5 mg (1.2 equivalents, 0.22 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 160 mg (1 equivalent, 0.18 mmol) of *N¹*-(Boc-^{L}Lys)-*N⁶*-[{6-(boc-^{L}Lys) amino} hexyl] hexane-1,6-diamine, **3a** dissolved in dry DCM (4 mL) was added dropwise to the reaction mixture. The reaction mixture was brought to room temperature and allowed to stir for 24 h. The solvent was evaporated, and the residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as an eluent to afford compound **3b** with 47% yield. ¹H-NMR (400 MHz, CDCl₃) δ/ppm: 7.949-7.929 (d, Ar*H*, 1H), 7.873-7.854 (d, Ar*H*, 1H), 7.850-7.777 (d, Ar*H*, 1H), 7.757-7.469 (m, Ar*H*, 2H), 7.432-7.393 (t, Ar*H*, 1H), 7.339-7.322 (d, Ar*H*, 1H), 6.472-6.495 (d, R-CO-N(-(CH₂)₆-N*H*-CO-^{L}Lys(Boc)₂)₂, 2H), 5.289-5.180 (d, R-CO-N(-(CH₂)₆-NH-CO-CH(N*H*Boc)(CH₂)₄-NHBoc)₂, 2H), 4.637 (br, R-CO-N(-(CH₂)₆-NH-CO-CH(NHBoc)(CH₂)₄-N*H*Boc)₂, 2H), 4.123 (s, Ar-C*H₂*-CO- N(-(CH₂)₆-NH-CO-CH(NHBoc)(CH₂)₄-N*H*Boc)₂, 2H), 4.005 (br, R-CO-N(-(CH₂)₆-NH-CO-*CH*(NHBoc)(CH₂)₄-NHBoc)₂, 2H), 3.375-3.079 (m, R-CO-N(-*CH₂*-(CH₂)₄-*CH₂*-NH-CO-CH(NHBoc)(CH₂)₃-*CH₂*-N*H*Boc)₂, 12H), 1.717-1.538 (m, *-CH₂-,* 20H), 1.433 (s, R-CO-N((CH₂)₆-NH-CO-CH(NH-COO-C*(CH₃)₃*)(CH₂)₄-NH-COO-C*(CH₃)₃*)₂, 36H), 1.346-1.219 (m, *-CH₂-,* 8H). HRMS (m/z): 1040.6816 [(M+H)⁺] (Observed), 1040.7011 [(M+H)⁺] (Calculated).

### N,N-bis-[(6-(^{L}Lys)amido)hexyl] naphthyl ethanamide tetrakistrifluoroacetate (Compound 13):

90 mg (1 equivalent, 0.087 mmol) of **3b** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 8.408-7.722 (m, Ar*H* and R-CO-N(-(CH₂)₆-N*H*-CO-CH(N*H₃*⁺)(CH₂)₄-N*H*₃⁺)₂, 18H), 7.527-7.324 (m, Ar*H*, 3H), 4.117 (s, Ar*CH₂*CO-N-(-(CH₂)₆-N*H*-CO-CH(N*H₃*⁺)(CH₂)₄-N*H₃*⁺)₂, 2H), 3.665 (br, R-CO-N(-(CH₂)₆-NH-CO-*CH*(NH₃⁺)(CH₂)₄-NH₃⁺)₂, 2H), 3.273-3.077 (m, R-CO-N(-(CH₂)₅-*CH₂*-NH-CO-CH(NH₃⁺)(CH₂)₃-*CH₂*-NH₃⁺)₂, 8H), 2.737 (br, R-CO-N(-*CH₂*-(CH₂)₅-NH-CO-CH(NH₃⁺)(CH₂)₃-CH₂-NH₃⁺)₂, 4H), 1.689-1.652 (m, R-CO-N(-(CH₂)₆-NH-CO-CH(NH₃⁺)(*CH₂*-(CH₂)₃- NH₃⁺)₂, 4H), 1.519-1.235 (m, R-CO-N(-CH₂-*(CH₂)₄*-CH₂-NH-CO-CH(NH₃⁺)(CH₂-*(CH₂)₂*-CH₂-NH₃⁺)₂, 24H). HRMS (m/z): 640.4956 [(M+H)⁺] (Observed), 640.4914 [(M+H)⁺] (Calculated).

### Example 10: Synthesis of N,N-bis-[(6-(^{L}Lys)amido) hexyl] 3,3-diphenyl propanamide tetrakis trifluoroacetate (Compound 14): (not part of the present invention)

### N,N-bis-[{6-(Boc-^{L}Lys)amido} hexyl] 3,3 diphenyl propanamide (3c):

233 mg (1.2 equivalents, 1.0 mmol) of 3,3-Diphenyl propionic acid was dissolved in dry DCM (4 mL) at 0 °C. In the reaction mixture, 448.9 µL (3 equivalents, 2.6 mmol) of *N,N*-diisopropylethylamine (DIPEA) was added followed by 390.63 mg (1.2 equivalents, 1.0 mmol) of *N,N,N',N'*-tetramethyl-O-(1H-benzotriazol-1-yl)uronium hexafluorophosphate (HBTU). Then DMF (2 mL) was added to the reaction mixture to dissolve the HBTU. After 15 min, 750 mg (1 equivalent, 0.859) of *N¹*-(Boc-^{L}Lys)-*N⁶*-[{6-(boc-^{L}Lys) amino} hexy1] hexane-1,6-diamine, **3a** dissolved in dry DCM (4 mL) was added dropwise to the reaction mixture. The reaction mixture was brought to room temperature and allowed to stir for 24 h. The solvent was evaporated, and the residue was diluted in ethyl acetate (50 mL). Then workup was carried out first with 1 N HCl (50 mL, 3 times) followed by saturated Na₂CO₃ solution (50 mL, 3 times). The crude product was extracted in ethyl acetate layer. Finally, purification was accomplished through column chromatography on silica gel (60-120 mesh) using different ratios of methanol and chloroform as an eluent to afford compound **3c** with 59% yield. ¹H-NMR (400 MHz, CDCl₃) δ/ppm: 7.275 (s, ArH, 2H), 7.240-7.147 (m, Ar*H*, 8H), 6.463-6.311 (d, R-CO-N(-(CH₂)₆-N*H*-CO-CH(NHBoc)(CH₂)₄-NHBoc)₂, 2H), 5.310-5.159 (d, R-CO-N(-(CH₂)₆-NH-CO-CH(N*H*Boc)(CH₂)₄-NHBoc)₂, 2H), 4.736-4.699 (t, Ar-*CH*-CH₂-N(-(CH₂)₆-NH-CO-CH(NHBoc)(CH₂)₄-NHBoc)₂, 1H), 4.640 (br, R-CO-N(-(CH₂)₆-NH-CO-C*H*(NHBoc)(CH₂)₄-N*H*Boc)₂, 2H), 4.108-3.995 (br, R-CO-N(-(CH₂)₆-NH-CO-CH(NHBoc)(CH₂)₄-N*H*Boc)₂, 2H), 3.232-3.088 (m, R-CO-N(-*CH₂*-(CH₂)₄-*CH₂*-NH-CO-CH(NHBoc)(CH₂)₃-*CH₂*-N*H*Boc)₂, 12H), 3.013-2.994 (d, Ar-CH-*CH₂*-N(-(CH₂)₆-NH-CO-CH(NHBoc)(CH₂)₄-N*H*Boc)₂, 2H), 1.823-1.561 (m, *-CH₂-,* 12H), 1.433 (s, R-CO-N((CH₂)₆-NH-CO-CH(NH-COO-C*(CH₃)₃*)(CH₂)₄-NH-COO-C*(CH₃)₃*)₂, 36H), 1.392-1.097 (m, *-CH₂-,* 16H). HRMS (m/z): 1080.7631 [(M+H)⁺] (Observed), 1080.7324 [(M+H)⁺] (Calculated).

550 mg (1 equivalent, 0.51 mmol) of **3c** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 8.459- 7.839 (m, R-CO-N(-(CH₂)₆-NH-CO-CH(N*H₃*⁺)(CH₂)₄-N*H₃*⁺)₂, 12H), 7.292-7.128 (m, Ar*H*, 10H), 5.155-5.133 (br, R-CO-N(-(CH₂)₆-N*H*-CO-CH(NH₃⁺)(CH₂)₄-NH₃⁺)₂, 2H), 4.534-4.498 (t, ArC*H*CH₂-CO- N(-(CH₂)₆-NH-CO-CH(NH₃⁺)(CH₂)₄-NH₃⁺)₂, 1H), 3.698-3.685 (d, R-CO-N(-(CH₂)₆-NH-CO-*CH*(NH₃⁺)(CH₂)₄-NH₃⁺)₂, 2H), 3.220-2.687 (m, ArCH*CH₂*-CO-N(*-CH₂*-(CH₂)₄*-CH₂*-NH-CO-CH(NH₃⁺)(CH₂)₃-C*H*₂-NH₃⁺)₂, 14H), 1.696-1.042 (m, R-CO-N(-CH₂-*(CH₂)₄*-CH₂-NH-CO-CH(NH₃⁺)(*(CH₂)₃*-CH₂-NH₃⁺)₂, 28H). HRMS (m/z): 680.5191 [(M+H)⁺] (Observed), 680.5227 [(M+H)⁺] (Calculated).

### Example 11: Synthesis of N-(adamantan-2-yl)-2-[N',N'-{bis-(6-^{L}Lys) amido)hexyl} amino] ethanamide pentakistrifluoroacetate (Compound 15): (not part of the present invention)

### N-(adamantan-2-yl)-2-[N',N'-{bis-(6-(Boc-^{L}Lys)amido)hexyl}amino] ethanamide (4a):

300 mg of *N*¹-(Boc-^{L}Lys)-*N⁶*-[{6-(boc-^{L}Lys)amido} hexyl] hexane-1,6-diamine, **3a** (1 equivalent, 0.344 mmol) in DMF. 71.29 mg of K₂CO₃ (1.5 equivalents, 0.52 mmol) was added to the reaction mixture. Then, 93.60 mg of N-(adamantan-2-yl)-2-bromoethanamide (1 equivalent, 0.34 mmol), dissolved in DMF was added to the reaction mixture. The reaction was stopped after 24 hours. The reaction mixture was then dissolved in ethyl acetate and work-up was performed with ice-cold water (3 times) to remove the DMF. The ethyl acetate layer was collected and the solvent evaporated to get the pure product with 52% yield. ¹H-NMR (400 MHz, CDCl₃) δ/ppm: 6.517 (br, Ad-NH-CO-CH₂-N(-(CH₂)₆-N*H-*CO-CH(NHBoc)(CH₂)₄-NHBoc)₂, 2H), 5.2913 (br, Ad-NH-CO-CH₂-N(-(CH₂)₆-NH-CO-CH(N*H*Boc)(CH₂)₄-NHBoc)₂, 2H), 4.699 (br, Ad-NH-CO-CH₂-N(-(CH₂)₆-NH-CO-CH(NHBoc)(CH₂)₄-N*H*Boc)₂, 2H), 4.144-4.033 (m, Ad*H* and Ad-NH-CO-CH₂-N(-(CH₂)₆-NH-CO-*CH*(NHBoc)(CH₂)₄-N*H*Boc)₂, 3H), 3.227-3.090 (br, Ad-NH-CO-*CH₂*-N(-(CH₂)s-*CH₂*-NH-CO-CH(NHBoc)(CH₂)₃-*CH₂*-N*H*Boc)₂, 10H), 2.039-1513 (m, Ad*H*, -CH₂-_{(Boc-Lys) amidohexyl} and -CH₂-_{(Lys)}, 34H), 1.430 (s, Ad-NH-CO-CH₂-N(-(CH₂)₅-CH₂-NH-CO-CH(NH-COO-C*(CH₃)₃*)(CH₂)₃-CH₂-NH-COO-C*(CH₃)₃*)₂, 36H), 1.357-1.236 (m, Ad*H*, -CH₂-_{(Boc-Lys) amidohexyl} and -CH₂-_{(Lys)}, 12H). HRMS (m/z): 1063.7691 [(M+H)⁺] (Observed), 1063.7746 [(M+H)⁺] (Calculated).

190 mg (1 equivalent, 0.18 mmol) of **4a** was dissolved in dry DCM. To the intensely stirred solution, 4 equivalents (excess amount) of trifluoroacetic acid (TFA) was added and allowed to stir at RT for 4-6 h. After that reaction, solvent and unused TFA were removed to get the pure product with 100% yield with CF₃COO⁻ counterions. The final compounds can be synthesized with other counterions such as Cl⁻, Br⁻ etc. ¹H-NMR (400 MHz, DMSO-d₆) δ/ppm: 9.509 (br, Ad-N*H*-CO-CH₂-N(-(CH₂)₆-NH-CO-CH(NH₃⁺)(CH₂)₄-NH₃⁺)₂, 1H), 8.480-8.457 (t, Ad-NH-CO-CH₂-N(-(CH₂)₆-N*H*-CO-CH(NH₃⁺)(CH₂)₄-NH₃⁺)₂, 2H), 8.149 (br, -NH₃*⁺,* 6H), 7.816 (br, -NH₃*⁺,* 6H), 3.975-3.899 (br, Ad-NH-CO-CH₂-N(-(CH₂)₆-NH-CO-*CH*(NH₃⁺)(CH₂)₄-NH₃⁺)₂, 2H), 3.820 (s, Ad-NH-CO-*CH₂*-N(-(CH₂)₆-NH-CO-CH(NH₃⁺)(CH₂)₄-NH₃⁺)₂, 2H), 3.696-3.685 (br, Ad*H*, 1H), 3.096-3.086 (br, Ad-NH-CO-CH₂-N(-(CH₂)₅-*CH₂*-NH-CO-CH(NH₃⁺)(CH₂)₃-*CH₂*-NH₃⁺)₂, 8H), 2.751 (br, Ad-NH-CO-CH₂-N(-*CH₂*-(CH₂)₅-NH-CO-CH(NH₃⁺)(CH₂)₃-CH₂-NH₃⁺)₂, 4H), 1.968-1.235 (m, Ad*H*, -CH₂-_{(Lys amidohexyl)} and -CH₂-_{(Lys)}, 42H). HRMS (m/z): 663.5619 [(M+H)⁺] (Observed), 663.5649 [(M+H)⁺] (Calculated).

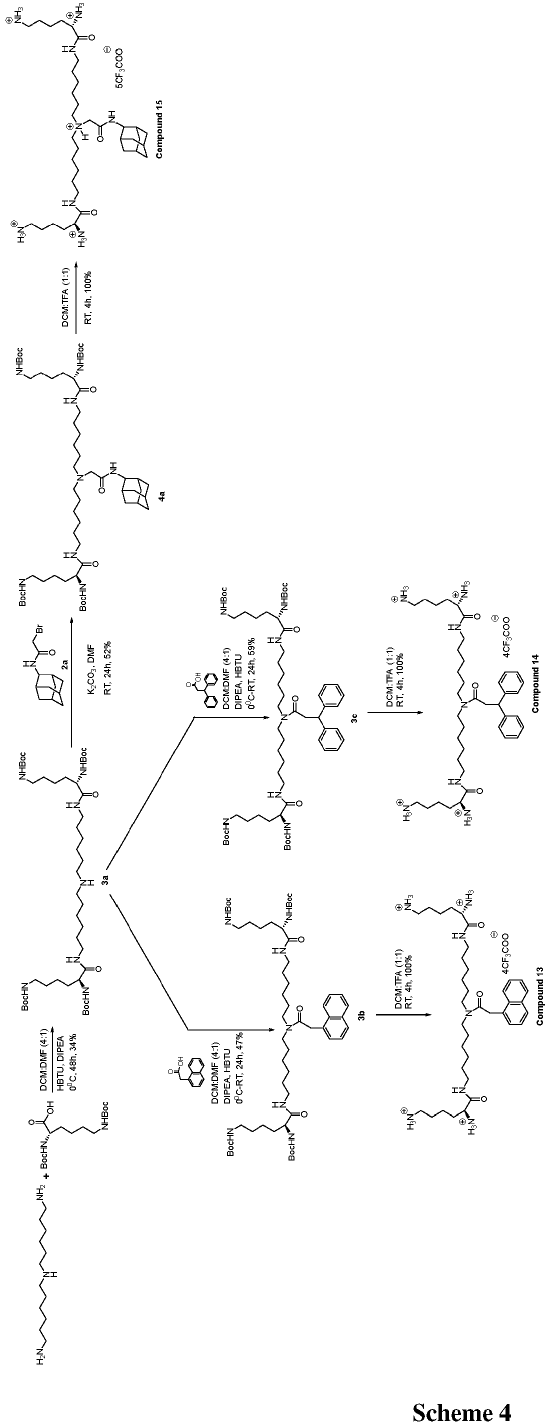

### Example 12: In-vitro Combination efficacy:

All synthesized compounds were assayed for their antibacterial activity in combination with antibiotics in a modified chequerboard micro-dilution broth format. Stock solutions were made by serially diluting the compounds using autoclaved millipore water. The bacterial freeze dried stock samples were stored at -80°C. About 3 µL of these stocks were added to about 3 mL of the respective broth and the culture was grown for about 6 h at about 37°C with prior to the experiments. This 6 h grown culture gives about 10⁸ cfu/mL in case of *A*. *baumannii* R674, *P. aeruginosa* R590, *E. coli* R3336 *and K. pneumoniae* R3934 which were determined by spread plating method. This 6 h grown culture was diluted to give effective cell concentration of 10⁵ cfu/mL, and then used for the chequerboard assay. Adjuvants and antibiotics were serially diluted in two-fold, in sterile millipore water. 25 µL of these serial dilutions of the antibiotics and 25 µL of compounds were added to the wells of 96 well plate followed by the addition of about 150 µL of bacterial solution (~10⁵ cfu/mL). The plates were then incubated at about 37°C, 150 rpm in the incubator and O.D at 600 nm was recorded at 24 h using TECAN (Infinite series, M200 pro) Plate Reader. The wells with similar OD as the media control contained the required concentration of the antibiotic and adjuvant for antibacterial efficacy. The potentiation ability of the adjuvants was determined by calculating the potentiation factor and the Fractional Inhibitory concentration (FIC) index. Fractional Inhibitory Concentration (FIC) is expressed as, FIC = [X]/MIC_{X}, where [X] is the lowest inhibitory concentration of antibiotic in the presence of the adjuvant. Whereas FIC index is expressed as, FICI = FIC_{antibiotic} + FIC_{adjuvant}. The values of FIC index give an insight about the nature of the combination. Potentiation factor which means fold-reduction in the MIC of the antibiotic, when used in combination with the adjuvant was also determined. Potentiation factor = [Minimum inhibitory concentration (MIC) of antibiotic in absence of adjuvant] / [MIC of antibiotic in presence of adjuvant]. The adjuvants exhibited decent to good potentiation of the antibiotics with potentiation factor as high as >1024. The combination efficacies of adjuvants with various antibiotics are furnished in Table 1. It should be noted that various other resistant strains of bacteria (Gram-positive and Gram-negative) can be tested with a number of other antibiotics to check the potentiation capacity of the adjuvants.

**Table 1: Combination efficacies of adjuvants with antibiotics against MDR strains of Gram-negative bacteria**

| Adjuvant | Bacterial strain | Antibiotic | MIC of antibiotic in presence of adjuvant (µg mL⁻¹) | Potentiation factor | FICI |
|---|---|---|---|---|---|
| **Compound 1** | *A*. *baumannii* R674 | Rifampicin | 32 | 2 | <0.75 |
| | | Tetracycline | 64 | 2 | <0.5 |
| **Compound 2*** | *A*. *baumannii* R674 | Rifampicin | 8 | 8 | <0.375 |
| | | Tetracycline | 64 | 2 | <0.625 |
| | *P. aeruginos a* R590 | Rifampicin | 16 | 8 | <0.375 |
| | | Tetracycline | 64 | 4 | <0.5 |
| **Compound 3*** | *A*. *baumannii* R674 | Rifampicin | 16 | 4 | <0.5 |
| | | Tetracycline | >64 | <2 | ND |
| | *P. aeruginos a* R590 | Rifampicin | 32 | 4 | <0.5 |
| | | Tetracycline | >64 | <4 | ND |
| **Compound 4** | *A*. *baumannii* R674 | Rifampicin | 1 | 64 | <0.26 |
| | | Tetracycline | 32 | 4 | <0.5 |
| | *P. aeruginos a* R590 | Rifampicin | 4 | 32 | <0.28 |
| | | Tetracycline | 64 | 4 | <0.5 |
| **Compound 5*** | *A*. *baumannii* R674 | Rifampicin | 8 | 8 | <0.375 |
| | | Tetracycline | >64 | <2 | ND |
| | *P. aeruginos a* R590 | Rifampicin | 8 | 16 | <0.3125 |
| | | Tetracycline | >64 | <4 | ND |
| **Compound 6** | *A*. *baumannii* R674 | Rifampicin | <0.25 | >256 | <0.06 |
| | | Tetracycline | >64 | <2 | ND |
| | *P. aeruginos a* R590 | Rifampicin | <0.25 | >512 | <0.06 |
| | | Tetracycline | >64 | <4 | ND |
| **Compound 7*** | *A*. *baumannii* R674 | Rifampicin | 8 | 8 | <0.25 |
| | | Tetracycline | 32 | 4 | <0.5 |
| | *P. aeruginos a* R590 | Rifampicin | 4 | 32 | <0.28 |
| | | Tetracycline | 64 | 4 | <0.5 |
| **Compound 8*** | *A*. *baumannii* R674 | Rifampicin | 4 | 16 | <0.19 |
| | | Tetracycline | >64 | <2 | ND |
| | | Rifampicn | 2 | 64 | <0.14 |
| | *P. aeruginos a* R590 | Tetracycline | 64 | 4 | <0.375 |
| **Compound 9*** | *A*. *baumannii* R674 | Rifampicin | 1 | 64 | <0.14 |
| | | Tetracycline | 16 | 8 | <0.25 |
| | *P. aeruginos a* R590 | Rifampicin | 0.5 | 256 | <0.13 |
| | | Tetracycline | 16 | 16 | <0.19 |
| **Compound 10** | *A*. *baumannii* R674 | Rifampicin | 1 | 64 | <0.14 |
| | | Tetracycline | 16 | 8 | <0.375 |
| | *P. aeruginos a* R590 | Rifampicin | 1 | 128 | <0.133 |
| | | Tetracycline | 32 | 8 | <0.375 |
| **Compound 11** | A. *baumannii* R674 | Rifampicin | 0.5 | 128 | 0.1328 |
| | | Tetracycline | 64 | 2 | 1 |
| | *P. aeruginos a* R590 | Rifampicin | 1 | 128 | 0.07 |
| | | Tetracycline | 32 | 8 | 0.375 |
| **Compound 12** | *A*. *baumannii* R674 | Rifampicin | <0.125 | >512 | <0.252 |
| | | Tetracycline | 16 | 8 | 0.625 |
| | *P. aeruginos a* R590 | Rifampicin | 0.125 | 1024 | 0.251 |
| | | Tetracycline | 64 | 4 | 0.75 |
| **Compound 13** | *A*. *baumannii* R674 | Rifampicin | 1 | 64 | 0.023 |
| | | Tetracycline | 16 | 8 | 0.375 |
| | | Minocycline | 0.5 | 16 | 0.1875 |
| | | Fusidic acid | <0.5 | >128 | <0.023 |
| | | Linezolid | 32 | 8 | 0.1875 |
| | | Vancomycin | 32 | 2 | 0.5625 |
| | | Ciprofloxaci n | 64 | 4 | 0.3125 |
| | | Erythromyci n | 16 | 32 | 0.156 |
| | | Ampicillin | No potentiation | - | - |
| | | Streptomycin | No potentiation | - | - |
| | *P. aeruginos a* R590 | Rifampicin | <0.5 | >256 | <0.019 |
| | | Tetracycline | 4 | 64 | 0.266 |
| | | Minocycline | 0.5 | 16 | 0.1875 |
| | | Fusidic acid | 2 | 32 | 0.094 |
| | | Linezolid | 32 | 8 | 0.1875 |
| | | Chloramphen icol | 32 | 4 | 0.375 |
| | | Colistin | No potentiation | - | - |
| | | Daptomycin | No potentiation | - | - |
| | *E. coli* R3336 | Rifampicin | 8 | 64 | <0.031 |
| | | Minocycline | 0.5 | 64 | <0.031 |
| | | Fusidic acid | 32 | >16 | <0.125 |
| | | Linezolid | No potentiation | - | - |
| | *K. pneumoni ae* R3934 | Rifampicin | 1 | 512 | <0.064 |
| | | Minocycline | 0.5 | 128 | <0.07 |
| | | Fusidic acid | 4 | 128 | <0.133 |
| | | Linezolid | 64 | >8 | <0.25 |
| **Compound 14** | *A*. *baumannii* R674 | Rifampicin | <0.125 | >512 | <0.017 |
| | | Tetracycline | 16 | 8 | <0.25 |
| | | Minocycline | 1 | 8 | <0.129 |
| | | Fusidic acid | <0.5 | >128 | <0.012 |
| | | Linezolid | 32 | 8 | <0.156 |
| | | Vancomycin | 16 | 4 | <0.3125 |
| | | Ciprofloxaci n | 64 | 4 | <0.281 |
| | | Erythromyci n | 16 | 32 | <0.0625 |
| | | Ampicillin | No potentiation | - | - |
| | | Streptomycin | No potentiation | - | - |
| | | Rifampicin | <0.125 | >1024 | <0.016 |
| | *P. aeruginos a* R590 | Tetracycline | 16 | 16 | <0.1875 |
| | | Minocycline | 1 | 8 | <0.156 |
| | | Fusidic acid | <0.25 | >256 | <0.012 |
| | | Linezolid | 32 | 8 | <0.156 |
| | | Chloramphen icol | 32 | 4 | <0.3125 |
| | | Colistin | 0.25 | 4 | <0.266 |
| | | Daptomycin | No potentiation | - | - |
| | *E. coli* R3336 | Rifampicin | 4 | 128 | <0.047 |
| | | Minocycline | <0.25 | >128 | <0.0156 |
| | | Fusidic acid | 16 | >32 | <0.0625 |
| | | Linezolid | No potentiation | - | - |
| | *K. pneumoni ae* R3934 | Rifampicin | 0.5 | 1024 | <0.126 |
| | | Minocycline | 0.5 | 128 | <0.1328 |
| | | Fusidic acid | 2 | 256 | <0.1289 |
| | | Linezolid | 64 | >8 | <0.1328 |
| **Compound 15** | *A*. *baumannii* R674 | Rifampicin | 0.25 | 256 | 0.1289 |
| | | Tetracycline | 2 | 64 | 0.5156 |
| | | Minocycline | 0.5 | 16 | 0.5625 |
| | | Fusidic acid | <0.5 | >128 | <0.07 |
| | | Linezolid | 32 | 8 | 1.125 |
| | | Vancomycin | 32 | 2 | 0.5625 |
| | | Ciprofloxaci n | 64 | 4 | 1.25 |
| | | Erythromyci n | 32 | 16 | 0.1875 |
| | | Ampicillin | No potentiation | - | - |
| | | Streptomycin | No potentiation | - | - |
| | *P. aeruginos a* R590 | Rifampicin | <0.125 | >1024 | <0.126 |
| | | Tetracycline | 2 | 128 | 0.5078 |
| | | Minocycline | 2 | 4 | 0.5 |
| | | Fusidic acid | <0.25 | >256 | <0.1289 |
| | | Linezolid | 64 | 4 | 0.2812 |
| | | Chloramphen icol | 32 | 4 | 1.25 |
| | | Colistin | 0.25 | 4 | 0.3125 |
| | | Daptomycin | No potentiation | - | - |
| | *E. coli* R3336 | Rifampicin | 16 | 32 | <0.0469 |
| | | Minocycline | 0.5 | 64 | <0.0469 |
| | | Fusidic acid | 32 | >16 | <0.1875 |
| | | Linezolid | No potentiation | - | - |
| | *K. pneumoni ae* R3934 | Rifampicin | 2 | 256 | <0.1289 |
| | | Minocycline | 0.5 | 128 | <0.07 |
| | | Fusidic acid | 8 | 64 | <0.0781 |
| | | Linezolid | No potentiation | - | - |

| | | | | | |
|---|---|---|---|---|---|
| *Not part of the present invention | | | | | |

**Table 2: Antibacterial efficacies and toxicity of Compounds 1 to 15**

| Adjuvants | MIC against clinical isolates of bacteria (µg/mL) or HC₅₀ (µg/mL) | | | | |
|---|---|---|---|---|---|
| | *A*. *baumannii* R674 | *P. aeruginosa* R590 | *E. coli* R3336 | *K. pneumoniae* R3934 | HC₅₀ |
| **Compound 1** | >512 | ND | ND | ND | >1024 |
| **Compound 2*** | >512 | >512 | ND | ND | >1024 |
| **Compound 3*** | >512 | >512 | ND | ND | >1024 |
| **Compound 4** | >512 | >512 | ND | ND | >1024 |
| **Compound 5*** | >512 | >512 | ND | ND | >1024 |
| **Compound 6** | 512 | 512 | ND | ND | >1024 |
| **Compound 7*** | >512 | >512 | ND | ND | >1024 |
| **Compound 8*** | >512 | >512 | ND | ND | >1024 |
| **Compound 9*** | >512 | >512 | ND | ND | >1024 |
| **Compound 10** | >512 | >512 | ND | ND | >1024 |
| **Compound 11** | 128 | 256 | ND | ND | >1024 |
| **Compound 12** | 64 | 64 | ND | ND | >1024 |
| **Compound 13** | 512 | 512 | >512 | >512 | >1024 |
| **Compound 14** | >512 | >512 | >512 | >512 | >1024 |
| **Compound 15** | 128 | 64 | >512 | >512 | >1024 |

| | | | | | |
|---|---|---|---|---|---|
| *Not part of the present invention | | | | | |

### Example 13: Determination of haemolytic activity.

Erythrocytes were isolated from freshly drawn, heparanized human blood and re-suspended to 5 vol% in PBS (pH 7.4). In a 96-well microtiter plate, 150 µl of erythrocyte suspension was added to 50 µl of serially diluted compound. Two controls were made, one without compound and other with 50 µl of 1 vol% solution of Triton X-100. The plate was incubated for 1 h at 37°C. The plate was then centrifuged at 3,500 *rpm* for 5 min, 100 µl of the supernatant from each well was transferred to a fresh 96-well microtiter plate, and A₅₄₀ was measured. Percentage of hemolysis was determined as (*A* - A₀)/(Aₜₒₜₐₗ -*A*₀) x 100, where *A* is the absorbance of the test well, *A*₀ the absorbance of the negative controls (without compound), and *A*ₜₒₜₐₗ the absorbance of 100% hemolysis wells (with Triton X-100), all at 540nm.

Toxicity studies of the compounds were done on freshly drawn human RBCs. Toxicity of the compounds were defined by their HC₅₀ values (**Table 2**), i.e. the concentration of compound at which 50% of the blood cells are lysed. All the compounds exhibited high HC₅₀ values and were highly non-toxic against human red blood cells.

### Example 14: Determination of Bactericidal time-kill kinetics:

Briefly, *A*. *baumannii* R674 and *P. aeruginosa* R590 were grown in nutrient broth at 37⁰C for 6h. Combinations of Compound 13 and rifampicin (4 µg/mL + 2 µg/mL and 8 µg/mL + 2 µg/mL) and rifampicin alone (32 µg/mL) were inoculated with aliquots of *A*. *baumannii* R674 suspended in fresh media at ~1.8 × 10⁵ CFU/mL. Similarly, combination of Compound 15 and fusidic acid (8 µg/mL + 1 µg/mL), Compound 15 (128 µg/mL) and fusidic acid (16 µg/mL) were inoculated with aliquots of *P. aeruginosa* R590 suspended in fresh media at ~1.8 × 10⁵ CFU/mL. After specified time intervals (0, 1, 2, 4, 6 and 12 h), 20 µL aliquots were serially diluted 10-fold in 0.9% saline, plated on sterile MacConkey agar plates and incubated at 37⁰C overnight. The viable colonies were counted the next day and represented as log₁₀ (CFU/mL) (**Figure 1**). Combination of rifampicin and Compound 13 could completely kill the bacteria in 2 h and combination of fusidic acid and Compound 14 could kill bacteria in 12 h.

## Claims

1. A compound, its stereoisomers, pharmaceutically acceptable salts, polymorphs, solvates and hydrates thereof, which is selected from a group consisting of:

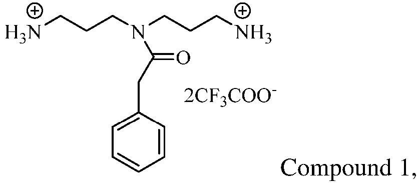

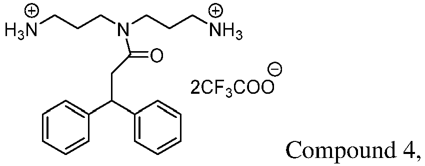

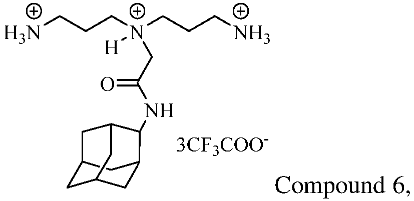

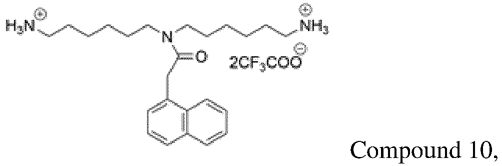

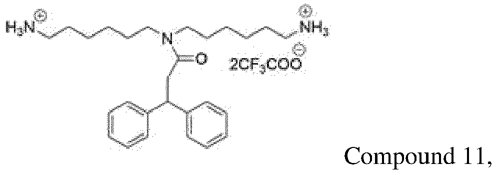

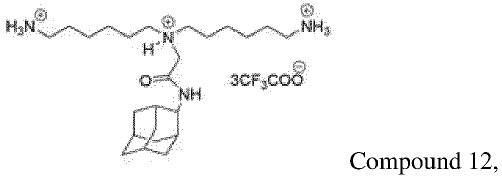

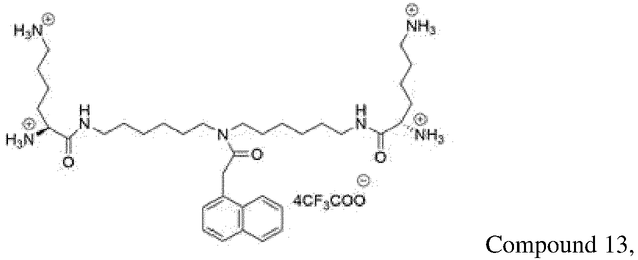

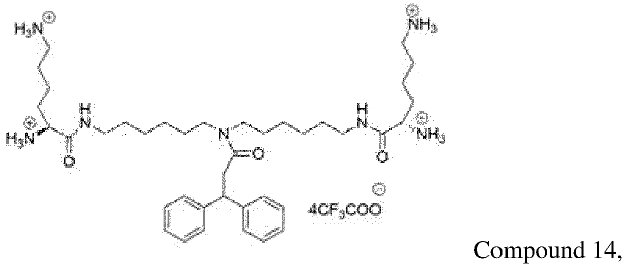

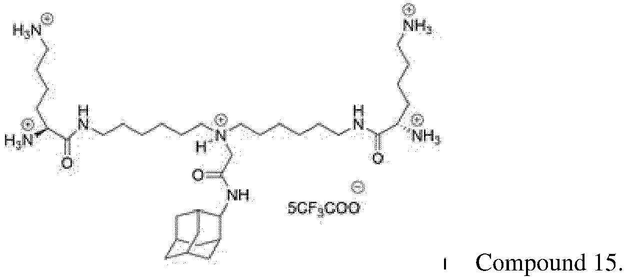

2. The compounds of claim 1 for use as small molecular adjuvants.

3. A pharmaceutical composition comprising a compound of claim 1 or its stereoisomers, pharmaceutically acceptable salts, polymorphs, solvates and hydrates thereof together with a pharmaceutically acceptable carrier, optionally in combination with one or more other antibiotics, or their pharmaceutical composition.

4. A pharmaceutical composition comprising:
a) a compound of claim 1 or its stereoisomers, pharmaceutically acceptable salts, polymorphs, solvates and hydrates; and
b) one or more antibiotics.

5. A pharmaceutical composition comprising:
a) a compound of claim 1 or its stereoisomers, pharmaceutically acceptable salts, polymorphs, solvates and hydrates thereof; and
b) a pharmaceutical composition of one or more antibiotics.

6. The pharmaceutical composition as claimed in any one of the claims 4 or 5, wherein the antibiotic is selected from the group consisting of rifampicin, tetracycline, fusidic acid, oxazolidinone, glycopeptides, fluoroquinolone, macrolide, beta-lactams, aminoglycosides, chloramphenicol, polymyxin, lipopeptide, and combinations thereof.

7. The pharmaceutical composition as claimed in any one of the claims 4 to 6, wherein the antibiotic is selected from the group consisting of rifampicin, tetracycline, minocycline, fusidic acid, linezolid, vancomycin, ciprofloxacin, erythromycin, ampicillin, streptomycin, chloramphenicol, colistin, daptomycin, and combinations thereof.

8. The pharmaceutical composition as claimed in any one of the claims 4 to 7, for use in treating disease or condition in a patient, wherein said disease or condition is caused by a microorganism selected from the group consisting of bacteria, fungi, and protozoa.

9. The pharmaceutical composition as claimed in any one of the claims 4 to 7, for use in killing or inhibiting the growth of a microorganism selected from the group consisting of bacteria, fungi, and protozoa.

10. The pharmaceutical composition for the use as claimed in any one of the claims 8 or 9, wherein the bacteria is Gram-positive bacteria or Gram-negative bacteria.

11. The pharmaceutical composition for the use as claimed in claim 10, wherein the bacteria is a multi-resistant bacterium selected from a group consisting of *A. baumannii, P. aeruginosa, E. coli, K. pneumoniae,* or any combinations thereof.

## Patentansprüche

1. Verbindung, ihre Stereoisomere, pharmazeutisch annehmbaren Salze, Polymorphe, Solvate und Hydrate davon, die ausgewählt sind aus einer Gruppe, bestehend aus:

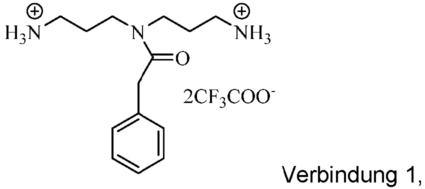

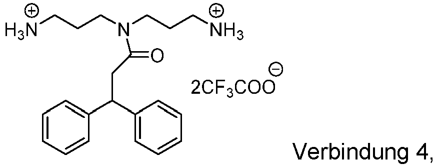

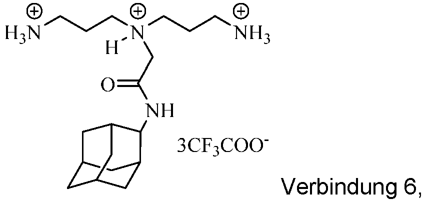

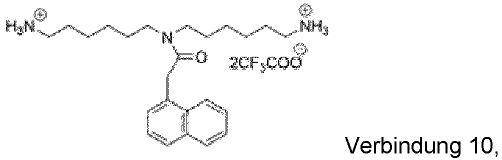

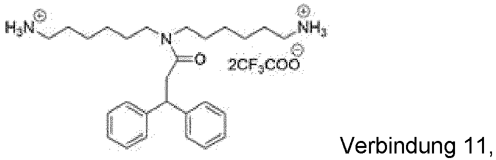

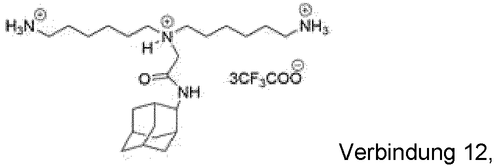

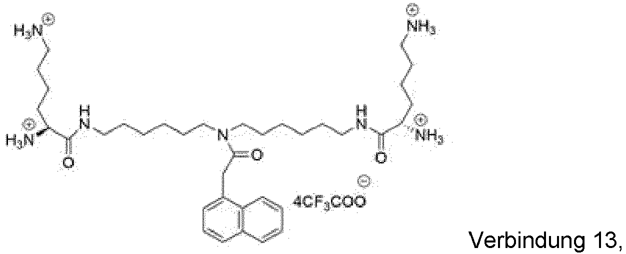

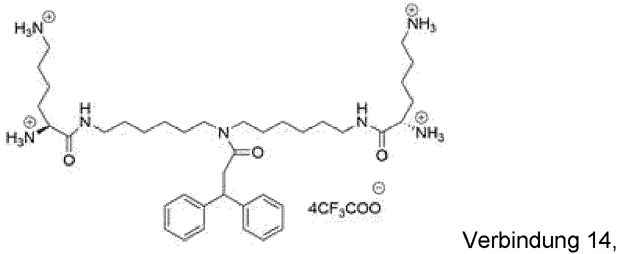

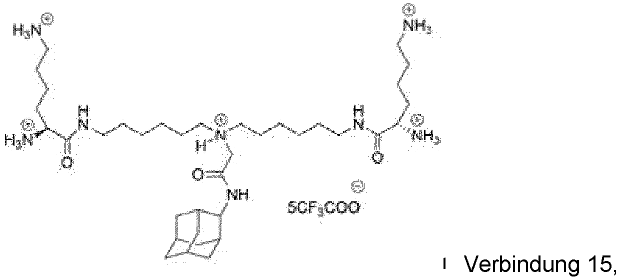

2. Verbindungen nach Anspruch 1 zur Verwendung als niedermolekulare Adjuvantien.

3. Pharmazeutische Zusammensetzung, umfassend eine Verbindung nach Anspruch 1 oder ihre Stereoisomere, pharmazeutisch annehmbaren Salze, Polymorphe, Solvate und Hydrate davon zusammen mit einem pharmazeutisch annehmbaren Träger, optional in Kombination mit einem oder mehreren anderen Antibiotika, oder ihre pharmazeutische Zusammensetzung.

4. Pharmazeutische Zusammensetzung, umfassend:
a) eine Verbindung nach Anspruch 1 oder ihre Stereoisomere, pharmazeutisch akzeptablen Salze, Polymorphe, Solvate und Hydrate; und
b) ein oder mehrere Antibiotika.

5. Pharmazeutische Zusammensetzung, umfassend:
a) eine Verbindung nach Anspruch 1 oder ihre Stereoisomere, pharmazeutisch annehmbaren Salze, Polymorphe, Solvate und Hydrate davon; und
b) eine pharmazeutische Zusammensetzung aus einem oder mehreren Antibiotika.

6. Pharmazeutische Zusammensetzung nach einem der Ansprüche 4 oder 5, wobei das Antibiotikum ausgewählt ist aus der Gruppe, bestehend aus Rifampicin, Tetracyclin, Fusidinsäure, Oxazolidinon, Glykopeptiden, Fluorchinolon, Makrolid, Beta-Lactamen, Aminoglycosiden, Chloramphenicol, Polymyxin, Lipopeptid und Kombinationen davon.

7. Pharmazeutische Zusammensetzung nach einem der Ansprüche 4 bis 6, wobei das Antibiotikum ausgewählt ist aus der Gruppe, bestehend aus Rifampicin, Tetracyclin, Minocyclin, Fusidinsäure, Linezolid, Vancomycin, Ciprofloxacin, Erythromycin, Ampicillin, Streptomycin, Chloramphenicol, Colistin, Daptomycin und Kombinationen davon.

8. Pharmazeutische Zusammensetzung nach einem der Ansprüche 4 bis 7 zur Verwendung bei der Behandlung einer Krankheit oder eines Zustands bei einem Patienten, wobei die Krankheit oder der Zustand durch einen Mikroorganismus, der ausgewählt ist aus der Gruppe, bestehend aus Bakterien, Pilzen und Protozoen, verursacht wird.

9. Pharmazeutische Zusammensetzung nach einem der Ansprüche 4 bis 7 zur Verwendung beim Abtöten oder Hemmen des Wachstums eines Mikroorganismus, der ausgewählt ist aus der Gruppe, bestehend aus Bakterien, Pilzen und Protozoen.

10. Pharmazeutische Zusammensetzung für die Verwendung nach einem der Ansprüche 8 oder 9, wobei die Bakterien um grampositive oder gramnegative Bakterien sind.

11. Pharmazeutische Zusammensetzung zur Verwendung nach Anspruch 10, wobei das Bakterium ein multiresistentes Bakterium ist, das ausgewählt ist aus der Gruppe, bestehend aus *A. baumannii, P. aeruginosa, E. coli, K. pneumoniae* oder beliebigen Kombinationen davon.

## Revendications

1. Composé, ses stéréo-isomères, ses sels pharmaceutiquement acceptables, ses polymorphes, ses solvates et ses hydrates, qui est choisi dans un groupe constitué par :

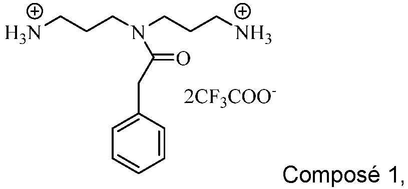

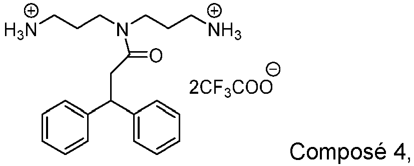

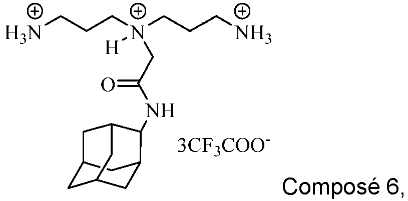

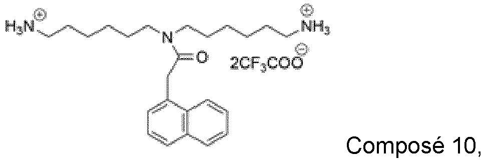

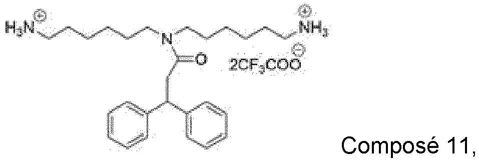

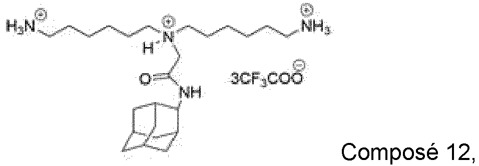

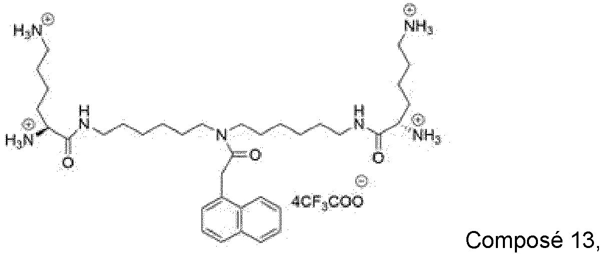

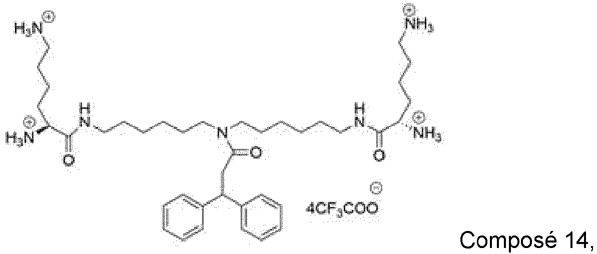

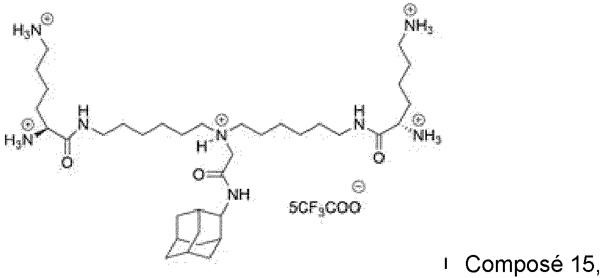

2. Composés selon la revendication 1 pour une utilisation en tant qu'adjuvants moléculaires de petite taille.

3. Composition pharmaceutique comprenant un composé selon la revendication 1 ou ses stéréo-isomères, ses sels pharmaceutiquement acceptables, ses polymorphes, ses solvates et ses hydrates, ainsi qu'un support pharmaceutiquement acceptable, facultativement en combinaison avec un ou plusieurs autres antibiotiques, ou leur composition pharmaceutique.

4. Composition pharmaceutique comprenant :
a) un composé selon la revendication 1 ou ses stéréo-isomères, ses sels pharmaceutiquement acceptables, ses polymorphes, ses solvates et ses hydrates ; et
b) un ou plusieurs antibiotiques.

5. Composition pharmaceutique comprenant :
a) un composé selon la revendication 1 ou ses stéréo-isomères, ses sels pharmaceutiquement acceptables, ses polymorphes, ses solvates et ses hydrates ; et
b) une composition pharmaceutique d'un ou plusieurs antibiotiques.

6. Composition pharmaceutique selon l'une quelconque des revendications 4 ou 5, dans laquelle l'antibiotique est choisi dans le groupe constitué par la rifampicine, la tétracycline, l'acide fusidique, l'oxazolidinone, les glycopeptides, la fluoroquinolone, les macrolides, les bêta-lactamines, les aminoglycosides, le chloramphénicol, la polymyxine, les lipopeptides et les combinaisons de ceux-ci.

7. Composition pharmaceutique selon l'une quelconque des revendications 4 à 6, dans laquelle l'antibiotique est choisi dans le groupe constitué par la rifampicine, la tétracycline, la minocycline, l'acide fusidique, le linézolide, la vancomycine, la ciprofloxacine, l'érythromycine, l'ampicilline, la streptomycine, le chloramphénicol, la colistine, la daptomycine et des combinaisons de ceux-ci.

8. Composition pharmaceutique selon l'une quelconque des revendications 4 à 7, pour une utilisation dans le traitement d'une maladie ou d'une affection chez un patient, dans laquelle ladite maladie ou affection est causée par un micro-organisme choisi dans le groupe constitué par les bactéries, les champignons et les protozoaires.

9. Composition pharmaceutique selon l'une quelconque des revendications 4 à 7, utilisée pour tuer ou inhiber la croissance d'un micro-organisme choisi dans le groupe constitué par les bactéries, les champignons et les protozoaires.

10. Composition pharmaceutique pour l'utilisation selon l'une quelconque des revendications 8 ou 9, dans laquelle les bactéries sont des bactéries à Gram positif ou des bactéries à Gram négatif.

11. Composition pharmaceutique pour l'utilisation visée selon la revendication 10, dans laquelle la bactérie est une bactérie multirésistante choisie dans le groupe constitué par *A. baumannii, P. aeruginosa, E. coli, K. pneumoniae,* ou de quelconques combinaisons de celles-ci.
